# EUROPEAN PATENT SPECIFICATION

(11) **EP 4 120 921 B1**
(45) Date of publication and mention of the grant of the patent: **25.06.2025**
(21) Application number: 21718707.9
(22) Date of filing: 19.03.2021
(51) Int. Cl.: A61B 17/00

(54) **MULTI-COMPONENT SEALANT DELIVERY SYSTEMS INCORPORATING QUARTER TURN CONNECTORS**
MEHRKOMPONENTEN-DICHTUNGSMITTELABGABESYSTEME MIT VIERTELDREHUNGSVERBINDERN
SYSTÈMES DE DISTRIBUTION DE PRODUIT D'ÉTANCHÉITÉ À COMPOSANTS MULTIPLES INCORPORANT DES RACCORDS QUART DE TOUR

(30) Priority: 20.03.2020 US 202062992191 P
(43) Date of publication of application: 25.01.2023
(73) Proprietor: Bard Peripheral Vascular, Inc., Franklin Lakes, NJ 07417 (US)
(72) Inventor: ADDISON, Jordan P., Franklin Lakes, NJ 07417 (US); BEYHL, Dylan, Franklin Lakes, NJ 07417 (US); BLANK, Ethan, Franklin Lakes, NJ 07417 (US); MOWREY, Nick, Franklin Lakes, NJ 07417 (US); STORM, Heather, A., Franklin Lakes, NJ 07417 (US); WINTERSTEIN, Luke, Franklin Lakes, NJ 07417 (US)
(74) Representative: Hoffmann Eitle
(86) International application number: PCT/US2021/023171
(87) International publication number: WO 2021/188904

(56) References cited:
- WO-A1-2009/086650
- WO-A1-2019/108618
- US-A- 5 104 375
- US-A- 5 116 315
- US-A1- 2004 254 533

## Description

### CROSS-REFERENCE TO RELATED APPLICATION

This application claims the priority benefit of U.S. Provisional Patent Application Serial No. 62,992,191, filed March 20, 2020 and entitled "SEALANT DELIVERY SYSTEM INCLUDING SEALANT APPLICATOR DEVICE, INJECTION NEEDLE ASSEMBLY, AND COMPONENT MIXER ASSEMBLY".

### BACKGROUND

### Field

The present disclosure generally relates to sealant delivery systems, and more specifically, to systems and methods for preparing the same, for use associated with a lung procedure to aid in preventing pneumothorax.

### Technical Background

Pneumothorax is a problematic complication of the lung biopsy procedure where air or fluid is allowed to pass into the pleural space as a result of the puncture of the parietal pleura and visceral pleura. Pneumothorax and, more so, pneumothorax requiring chest tube placement, are significant concerns for clinicians performing, and patients undergoing, percutaneous lung biopsies. The incidence of pneumothorax in patients undergoing percutaneous lung biopsy has been reported to be anywhere from 9-54%, with an average of around 15%. On average, 6.6% of all percutaneous lung biopsies result in pneumothorax requiring a chest tube to be placed, which results in an average hospital stay of 2.7 days.

Factors that increase the risk of pneumothorax include increased patient age, obstructive lung disease, increased depth of a lesion, multiple pleural passes, increased time that an access needle lies across the pleura, and traversal of a fissure. Pneumothorax may occur during or immediately after the procedure, which is why typically a CT scan of the region is performed following removal of the needle. Other, less common, complications of percutaneous lung biopsy include hemoptysis (coughing up blood), hemothorax (a type of pleural effusion in which blood accumulates in the pleural cavity), infection, and air embolism.

It has been suggested that approximately 30% of lung biopsies result in some form of pneumothorax that makes deploying a plug after the biopsy difficult or impossible. Current sealants on the market are deployed through a coaxial cannula after a biopsy is performed which may be before or after a pneumothorax forms.

United States Patent US 5,116,315 discloses biological syringe has a manifold with inlets for first and second fluids e.g. from syringes. The manifold has separate channels for the fluids and is connected to a discharge assembly. The discharge assembly mixes and delivers the fluids in a spray. The discharge assembly has separate conduits and a mixing chamber pref. having a shaped spray nozzle providing swirling flows.

International Patent Application WO 2009/086650 A1 discloses a dispensing appliance for a double cartridge comprising a housing for receiving multiple components and a rotatable portion having a thread, the two parts cooperating in such a manner that by rotating the rotatable portion, a multiple plunger acting upon the pistons in the housing is continuously displaceable relative to the housing in the dispensing direction.

### SUMMARY

In one aspect, a sealant delivery system includes a sealant applicator that includes two chambers separate from one another. Each chamber includes at least one output port on a distal end thereof. The sealant applicator further includes a quarter turn connector disposed on a distal end of the sealant applicator adjacent to the at least one output port of each chamber. The quarter turn connector is shaped to releasably interlock with a corresponding quarter turn connector of an injection needle assembly comprising a plurality of input ports or with a corresponding quarter turn connector of a dual chamber mixing syringe comprising a plurality of mixing ports. When the injection needle assembly or the dual chamber mixing syringe is coupled to the sealant applicator via the quarter turn connector, the plurality of input ports or the plurality of mixing ports are aligned and sealed with the at least one output port of each chamber of the sealant applicator. The quarter turn connector comprises a circular protrusion extending distally from the distal end of the sealant applicator, the circular protrusion comprising the at least one output port of each of the two chambers; a semi-circular channel disposed within the distal end of the sealant applicator along a periphery of the circular protrusion; and a pair of bayonet coupling members disposed radially outward of the circular protrusion and the semi-circular channel.

In another aspect, an injection needle assembly includes a hub having a hub quarter turn connector that releasably interlocks with a corresponding quarter turn connector disposed on a sealant applicator, a plurality of input ports disposed within the hub, and an elongate hollow stylet that extends distally from the hub. The elongate hollow stylet has a proximal portion at the hub and a distal portion spaced apart from the proximal portion. The elongate hollow stylet includes an outer side wall extending from the proximal portion to the distal portion and defining an outer lumen that is fluidly coupled to at least a first one of the plurality of input ports, an inner side wall extending from the proximal portion to the distal portion and defining an inner lumen disposed within the outer lumen such that the inner lumen is concentric with the outer lumen and has a cross-sectional size that is smaller than the outer lumen. The inner lumen is fluidly coupled to at least a second one of the plurality of input ports. The elongate hollow stylet further includes a mixing chamber disposed at the distal portion of the elongate hollow stylet. The mixing chamber is fluidly coupled to the outer lumen and the inner lumen and comprising at least one side port. When the hub quarter turn connector that interlocks with the quarter turn connector disposed on the distal end of the sealant applicator, the plurality of input ports are each aligned and sealed with a corresponding output port of a sealant applicator. The hub quarter turn connector comprises a circular recess sized to receive a circular protrusion on a distal end of the sealant applicator, the circular recess comprising the plurality of input ports disposed therein; a lip extending radially outward of the circular recess, the lip shaped and sized to be received within a pair of bayonet coupling members disposed on the sealant applicator; and a quarter circular boss extending proximally from the lip, the quarter circular boss shaped to be received within a semi-circular channel of the sealant applicator and slidable within the semi-circular channel in a quarter turn motion from an unlocked state to a locked state

Additional features and advantages of the aspects described herein will be set forth in the detailed description which follows, and in part will be readily apparent to those skilled in the art from that description or recognized by practicing the aspects described herein, including the detailed description, which follows, the claims, as well as the appended drawings.

It is to be understood that both the foregoing general description and the following detailed description describe various aspects and are intended to provide an overview or framework for understanding the nature and character of the claimed subject matter. The accompanying drawings are included to provide a further understanding of the various aspects, and are incorporated into and constitute a part of this specification. The drawings illustrate the various aspects described herein, and together with the description serve to explain the principles and operations of the claimed subject matter.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, wherein like structure is indicated with like reference numerals and in which:
FIG. 1 depicts a front view of an illustrative sealant delivery system including a sealant applicator, a dual chamber mixing syringe, and an injection needle assembly according to one or more aspects shown and described herein;
FIG. 2A depicts a front view of an illustrative mixing apparatus including a sealant applicator and a dual chamber mixing syringe according to one or more aspects shown and described herein;
FIG. 2B depicts a front view of an illustrative delivery apparatus including a sealant applicator and an injection needle assembly according to one or more aspects shown and described herein;
FIG. 3A depicts a perspective view of an illustrative quarter turn connector of a sealant applicator according to one or more aspects shown and described herein;
FIG. 3B depicts a distal view of the illustrative quarter turn connector of the sealant applicator of FIG. 3A;
FIG. 4A depicts a perspective view of an illustrative corresponding quarter turn connector of a dual chamber mixing syringe according to one or more aspects shown and described herein;
FIG. 4B depicts a perspective view of an illustrative corresponding quarter turn connector of an injection needle assembly according to one or more aspects shown and described herein;
FIG. 4C depicts a top view of the illustrative corresponding quarter turn connector of the injection needle assembly of FIG. 4B;
FIG. 4D depicts a section view of a distal portion of an illustrative injection needle assembly according to one or more aspects shown and described herein;
FIG. 5A depicts a section view of an interface of a quarter turn connector of a sealant applicator with a corresponding quarter turn connector of an injection needle assembly at an initial orientation according to one or more aspects shown and described herein;
FIG. 5B depicts a section view of the interface of FIG. 5A during a rotating motion;
FIG. 5C depicts a section view of the interface of FIG. 5A once the rotating motion of FIG. 5B has been completed;
FIG. 6A depicts a section view of the mixing apparatus of FIG. 2A showing an alignment of components according to one or more aspects shown and described herein;
FIG. 6B depicts a section view of the delivery apparatus of FIG. 2B showing an alignment of components according to one or more aspects shown and described herein;
FIG. 7A depicts a perspective view of another illustrative quarter turn connector of a sealant applicator according to one or more aspects shown and described herein;
FIG. 7B depicts a distal view of the other illustrative quarter turn connector of the sealant applicator of FIG. 7A;
FIG. 8A depicts a perspective view of another illustrative corresponding quarter turn connector of an injection needle assembly according to one or more aspects shown and described herein;
FIG. 8B depicts a distal view of the other illustrative corresponding quarter turn connector of FIG. 8A;
FIG. 8C depicts a side view of the other illustrative corresponding quarter turn connector of FIG. 8A;
FIG. 9A is a perspective view of yet another illustrative hub of a needle assembly according to one or more aspects shown and described herein;
FIG. 9B is a section view of the hub of FIG. 9A;
FIG. 10A depicts separate components of an illustrative clip fit connection according to one or more aspects shown and described herein;
FIG. 10B depicts the separate components of the clip fit connection joined together according to one or more aspects shown and described herein; and
FIG. 11 is a flow diagram of an illustrative method of preparing the various sealant delivery systems according to one or more aspects shown and described herein.

### DETAILED DESCRIPTION

The present disclosure, in one form, is related to sealant delivery systems for a lung access procedure, in particular for prevention of pneumothorax. The sealant delivery systems described herein include components that deliver the multicomponent sealant along an access path, including the pleura region, to the lung prior to performing a lung procedure, such as a biopsy or the like. The sealant delivery systems described herein include a dual chamber sealant applicator, a dual chamber mixing syringe, and an injection needle assembly. The sealant delivery systems described herein are configured such that a user can quickly and easily couple the dual chamber sealant applicator with the dual chamber mixing syringe in a manner that correctly aligns the applicator and mixing syringe so that the components within each can be mixed together. Once mixed, the user can just as easily disconnect the applicator from the mixing syringe and quickly and easily attach the injection needle assembly thereto for the purposes of delivering the sealant to a subject. To achieve this, the dual chamber sealant applicator includes a quarter turn connector that allows for the quick joining and releasing of the dual chamber mixing syringe and the injection needle assembly, which both have a corresponding quarter turn connector. In addition, because the sealant materials located in the dual chamber sealant applicator quickly form a sealant product when combined together, it is necessary to ensure the combination occurs at the side where the sealant product is to be distributed. As such, the needle assembly described herein includes an elongate hollow stylet having a mixing chamber at a distal end thereof, and utilizes an inner lumen disposed concentrically within an outer lumen to define separate passageways extending from the two chambers of the applicator to the mixing chamber at the distal end.

It should be understood that the term "quarter term" as used herein refers to a rotation that completes about one fourth of a full rotation. That is, a quarter turn rotation rotates about 90° clockwise or counterclockwise. The components described herein are referred to as "quarter turn" components because the components allow about a 90° rotation of components relative to one another.

An advantage of the present disclosure is that the various aspects described herein improve upon typical solutions in that the multi-component sealant seals the pneumothorax region before the biopsy, rather than after the biopsy.

Another advantage of the present disclosure is that the various aspects described herein require no measuring of where the needle is in relation to the pleura beyond what physicians currently do. The solution of the present disclosure should be able to be seamlessly integrated into the lung access procedure by preparation of the flowable multi-component sealant, and dispensing the flowable multi-component sealant from the elongate hollow stylet of the injection needle assembly as the elongate hollow stylet is advanced across the pleura.

Another advantage of the present disclosure is that the various quarter turn connectors that are used to couple components together provide a quick and easy way for a user to connect and disconnect components in a manner that ensures that the components are adequately aligned and sealed each time they are connected, thereby ensuring the correct mixture and delivery of materials. In addition, an advantage of the quarter turn connectors of the present disclosure is that the quarter turn connectors provide tactile or audible feedback provided to a user as extra confirmation that the components are correctly aligned and joined when coupled via the quarter turn connector.

Another advantage of the present disclosure is that the various aspects described herein improve over typical pneumothorax prevention devices in that, since the injection needle assembly is deployed at the beginning of the procedure rather than at the very end of a procedure, the flowable multi-component sealant, e.g., polymer, is able to be delivered and integrate into the spaces between tissues, whereas polymer plugs, for example, only occupy the space that they were cast and thus may result in a less effective seal.

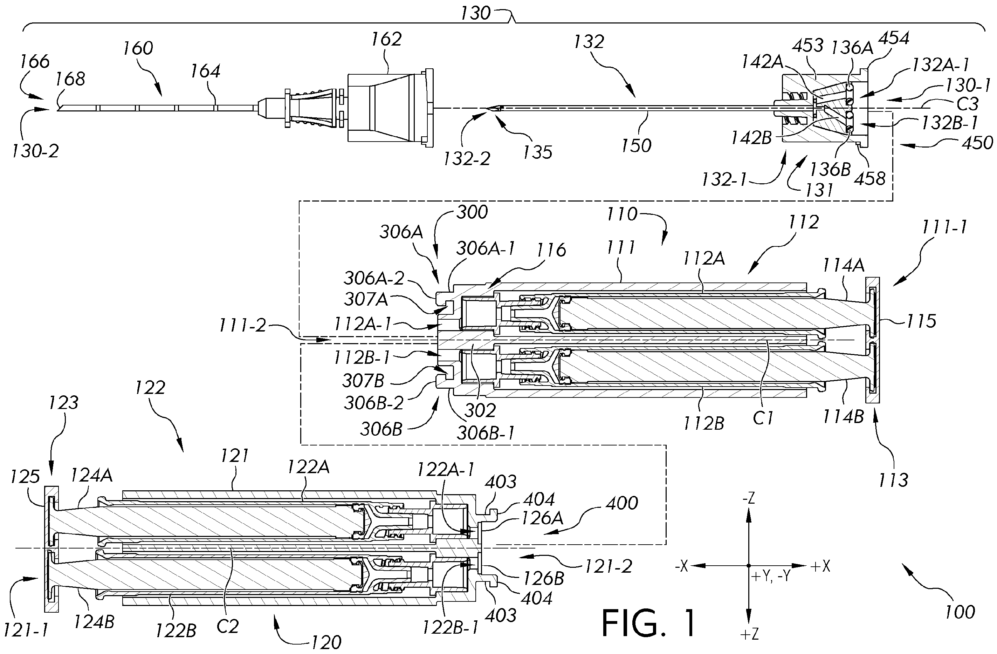

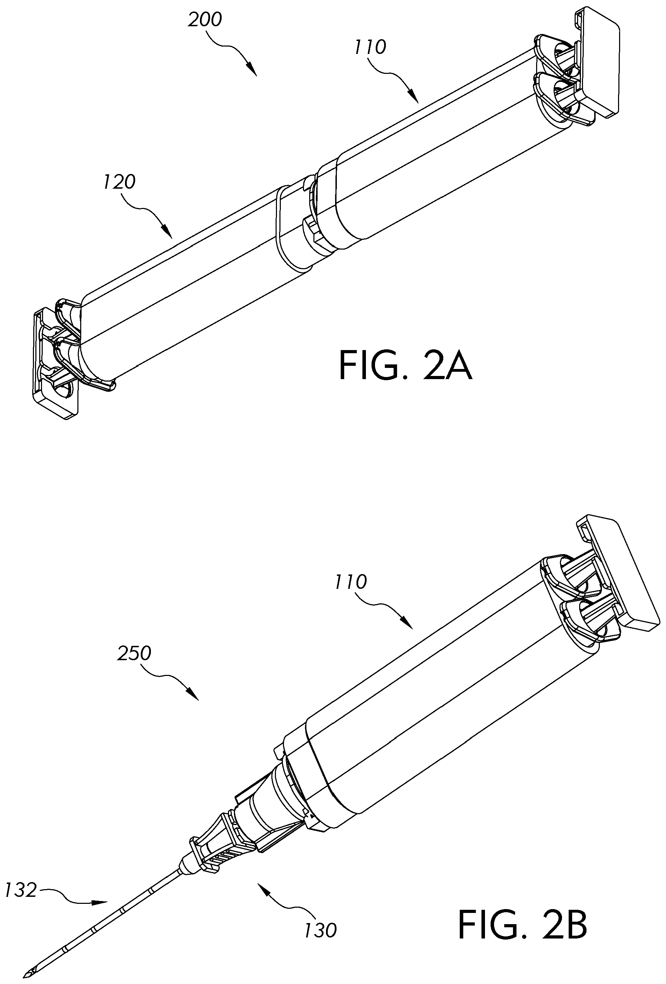

Turning now to the drawings, FIG. 1 depicts an illustrative sealant delivery system 100 according to various embodiments. The sealant delivery system 100, in accordance with an aspect of the present invention, may be for use in a lung access procedure to aid in preventing pneumothorax. The sealant delivery system 100 generally includes a sealant applicator 110, a dual chamber mixing syringe 120, and/or an injection needle assembly 130. The various components for the sealant delivery system 100 are keyed via a quarter turn connection system such that the sealant applicator 110 can be coupled to the dual chamber mixing syringe 120 for mixing components of a multi-component sealant, removed from the dual chamber mixing syringe 120, and coupled to the injection needle assembly 130 for further mixing and delivery of components, as indicated by the dashed lines in FIG. 1 and described herein. When coupled to the dual chamber mixing syringe 120, the combination of the dual chamber mixing syringe 120 and the sealant applicator 110 may be referred to herein as a mixing apparatus 200, as depicted in FIG. 2A. When coupled to the injection needle assembly 130, the combination of the injection needle assembly 130 and the sealant applicator 110 may be referred to herein as a delivery apparatus 250, as depicted in FIG. 2B.

Referring to FIG. 2B, when the sealant applicator 110 is coupled to the injection needle assembly 130, the delivery apparatus 250 therefrom has a configuration to facilitate delivery of a flowable multi-component sealant to an injection site. The multi-component sealant may be injected while the needle portion of the injection needle assembly 130 crosses the two layers of the pleura, (e.g., the parietal and visceral pleura). The injection needle assembly 130 is configured to puncture tissue and create an access path (e.g., a proposed biopsy tract) in the tissue at least through the pleura of the subject, and thus allows for the two layers of the pleura to be sealed (e.g., prior to a lung biopsy), by the curing of the multi-component sealant at the delivery site so that air cannot leak between the two layers and cause a pneumothorax. It is noted that the multi-component sealant also may be deposited in other regions of the access path, such as in the subcutaneous tissue and/or lung parenchyma.

Referring to FIGS. 1 and 2B, optionally, the delivery apparatus 250 may be used in conjunction with an introducer cannula 160. The introducer cannula 160 may facilitate withdrawal of the injection needle assembly 130 of the delivery apparatus 250 from a subject, while the introducer cannula 160 may remain in place to maintain the access path in the tissue to the site, such as for example, to receive and guide a second medical instrument, such as a biopsy device, to the site where the biopsy is to be performed.

Referring again to FIG. 1, the sealant applicator 110 generally includes a body 111 having a proximal end 111-1 and a distal end 111-2 spaced a distance apart from the proximal end 111-1. The body 111 also defines a pair of syringes 112. The sealant applicator 110 is configured to separately carry each of a first sealant component of the multi-component sealant and a second sealant component of the multi-component sealant. The first sealant component may include, for example, at least two N-hydroxysuccinimide (NHS) ester groups, and the second sealant component may include, for example, at least two amine groups. For example, the first sealant component may be a solution containing polyethylene glycol (PEG) succinimidyl succinate and the second sealant component may be a solution containing albumin and/or polyethylenimine (PEI). In the present embodiment, the first sealant component and the second sealant component are combined and mixed within a mixing chamber of the injection needle assembly 130, as will be described in greater detail herein.

The pair of syringes 112 includes an actuator 113, a first component chamber 112A, and a second component chamber 112B. The first component chamber 112A may be, for example, a cylindrical tube that is configured to carry the first sealant component of the multi- component sealant. The first component chamber 112A has a first output port 112A-1 (e.g., a first component port). The second component chamber 112B also may be, for example, a cylindrical tube that is configured to carry the second sealant component of the multi-component sealant. The second component chamber 112B has a second output port 112B-1 (e.g., a second component port). In some aspects, the first component chamber 112A and the second component chamber 112B are arranged in a substantially longitudinally parallel arrangement.

In some aspects, the actuator 113 includes a first piston 114A, a second piston 114B, and a handle 115. The handle 115 is in the form of a link member that perpendicularly extends between, and is connected to, each of the first piston 114A and the second piston 114B to facilitate simultaneous movement of the first piston 114A and the second piston 114B with the depression or retraction of the handle 115. The first piston 114A is in the form of a plunger that is positioned in the first component chamber 112A proximal to the first sealant component, and the second piston 114B is in the form of a plunger that is positioned in the second component chamber 112B proximal to the second sealant component.

The first output port 112A-1 of the first component chamber 112A and the second output port 112B-1 of the second component chamber 112B may be arranged within the distal end 111-2 of the body 111 of the sealant applicator 110. The first output port 112A-1 and the second output port 112B-1 are generally fluid outputs that are aligned with other ports of other components as described herein such that the first and second sealant components can be dispensed from and/or received within the respective component chambers 112A, 112B. In some aspects, the first output port 112A-1 may be concentrically aligned with the first component chamber 112A and the second output port 112B-1 may be concentrically aligned with the second component chamber 112B. However, in other aspects, such as the aspect depicted in FIG. 1, the first output port 112A-1 may be located radially inward of a central area of the first component chamber 112A and the second output port 112B-1 may be located radially inward of a central area of the second component chamber 112B such that the first output port 112A-1 and the second output port 112B-1 are as close as possible to a center axis C1 of the body 111 of the sealant applicator 110 to facilitate alignment with the other components of the sealant delivery system 100 described herein.

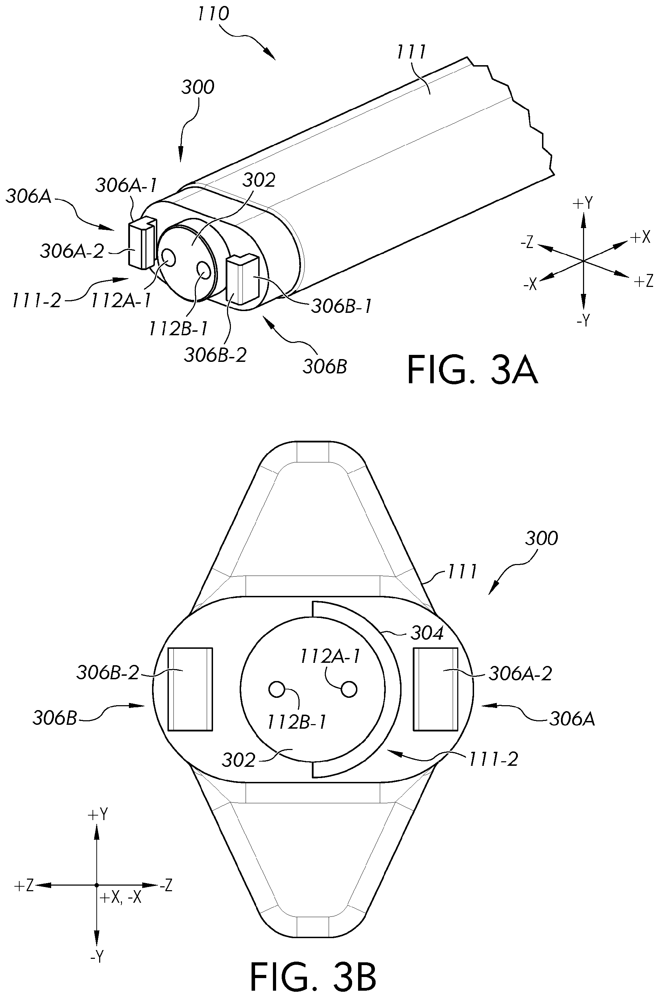

Turning to FIGS. 1, 3A, and 3B, the body 111 of the sealant applicator 110 further includes a quarter turn connector 300 integrated with the distal end 111-2 of the sealant applicator 110. More specifically, as depicted in FIG. 1, the various components of the quarter turn connector 300 are integrated with the body 111 such that the quarter turn connector 300 and the body 111 are a single monolithic piece. However, it should be understood that this is merely illustrative and the various components of the quarter turn connector 300 may be separate pieces that are permanently or semi-permanently joined with the body 111 of the sealant applicator 110 (e.g., permanently or semi-permanently joined with a distal coupling piece 116 of the sealant applicator 110).

The quarter turn connector 300 is generally located at the distal end 111-2 of the body 111 of the sealant applicator such that various components of the quarter turn connector 300 are positioned adjacent to first output port 112A-1 and the second output port 112B-1. As will be described herein, the quarter turn connector 300 is generally shaped and sized to releasably interlock with a corresponding quarter turn connector 400 of the dual chamber mixing syringe 120 and/or with a corresponding quarter turn connector 450 of the injection needle assembly 130. As will be described in greater detail herein, when the injection needle assembly 130 or the dual chamber mixing syringe 120 is coupled to the sealant applicator 110 via the quarter turn connectors 300, 400, 450 thereof, the various ports thereof are aligned and sealed with the first output port 112A-1 and the second output port 112B-1 of the sealant applicator 110.

Still referring to FIGS. 1, 3A, and 3B, the quarter turn connector 300 of the sealant applicator includes a circular protrusion 302 extending distally (e.g., in the -x direction of the coordinate axes of FIG. 3A) from the distal end 111-2 of the sealant applicator 110, a semi-circular channel 304 (FIG. 3B) disposed within the distal end 111-2 of the sealant applicator 110 along a periphery of the circular protrusion 302, and a pair of bayonet coupling members (e.g., a first coupling member 306A and a second coupling member 306B) disposed radially outward of the circular protrusion 302 and the semi-circular channel 304. As shown in FIGS. 1, 3A, and 3B, the first output port 112A-1 and the second output port 112B-1 are disposed within the circular protrusion 302. That is, the openings into the first component chamber 112A and the second component chamber 112B are located on the circular protrusion 302.

The circular protrusion 302 is generally shaped and sized to correspond to a recess formed in the injection needle assembly 130 and the dual chamber mixing syringe 120, as described in greater detail herein. The circular protrusion may generally be disposed in or around a central area of the distal end 111-2 of the body 111. In some embodiments, the circular protrusion 302 may be concentric with the body such that the center axis C1 of the body 111 extends through a center of the circular protrusion 302. The distance that the circular protrusion 302 extends away from the distal end 111-2 of the body is generally a distance that corresponds to a depth of the recess formed in the injection needle assembly 130 and the dual chamber mixing syringe 120 such that the circular protrusion 302 can be completely inserted therein, but is otherwise not limited by the present disclosure.

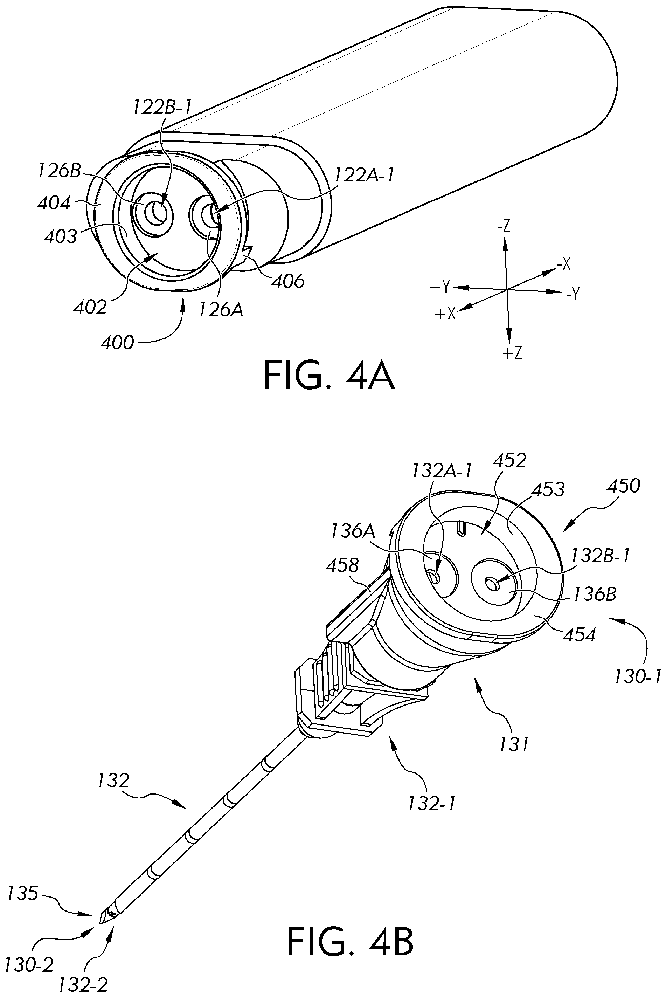

As particularly depicted in FIGS. 3A-3B, the semi-circular channel 304 is disposed around the periphery of the circular protrusion 302 such that the semi-circular channel 304 defines an outer edge of half of the circular protrusion 302. That is, the semi-circular channel 304 extends from a first side (e.g., in the +y direction of the coordinate axes of FIGS. 3A-3B) around the perimeter of the circular protrusion 302 to a second side (e.g., in the -y direction of the coordinate axes of FIGS. 3A-3B). The semi-circular channel 304 is generally shaped and sized to receive a quarter circular extension 456 (FIG. 4A) of the corresponding quarter turn connector 400 of the dual chamber mixing syringe 120 (FIG. 1) or the quarter circular extension 456 of the corresponding quarter turn connector 450 of the injection needle assembly 130, as described in greater detail herein. While the aspects depicted in FIGS. 3A-3B depict the semi-circular channel 304 as extending a full semi-circle, the present disclosure is not limited to such. That is, the semi-circular channel 304 may completely surround the circular protrusion 302 in other aspects (e.g., a full circular channel) or may only surround a portion of the circular protrusion 302 to an extent that is lesser than or greater than the semi-circular aspect depicted in FIGS. 3A-3B (e.g., a quarter circular channel or the like). However, it should be understood that, to ensure appropriate keying such that the various components of the sealant delivery system 100 (FIG. 1) are appropriately aligned and rotatable via the respective quarter turn connectors 300, 400, 450, the channel corresponds in size to the size of the extensions.

Referring again to FIGS. 1, 3A, and 3B, the first coupling member 306A and the second coupling member 306B each extend from the distal end 111-2 of the body 111 of the sealant applicator 110 and are generally shaped and sized to retain the dual chamber mixing syringe 120 or the injection needle assembly 130 when coupled to the sealant applicator 110. Each of the first coupling member 306A and the second coupling member 306B may be a bayonet style coupling member, an L-beam coupling member, or the like. For example, as particularly depicted in FIG. 1, the first coupling member 306A extends distally at a particular distance from the distal end 111-2 of the body 111, turns about 90 degrees, and extends inward toward the center axis C1 of the body 111, resulting in a first extension piece 306A-1 that extends in a direction generally coplanar with the center axis C1 of the body 111 (e.g., along the x-axis of the coordinate axes of FIGS. 1, 3A, and 3B) and a second extension piece 306A-2 that extends in a direction that is generally perpendicular to the center axis C1 of the body (e.g., along the z-axis of the coordinate axes of FIGS. 1, 3A, and 3B), thereby defining a pocket 307A between the distal end 111-2 of the body 111 and the second extension piece 306A-2. Similarly, the second coupling member 306B extends distally at a particular distance from the distal end 111-2 of the body 111, turns about 90 degrees, and extends inward toward the center axis C1 of the body 111, resulting in a first extension piece 306B-1 that extends in a direction generally coplanar with the center axis C1 of the body 111 (e.g., along the x-axis of the coordinate axes of FIGS. 1, 3A, and 3B) and a second extension piece 306B-2 that extends in a direction that is generally perpendicular to the center axis C1 of the body 111 (e.g., along the z-axis of the coordinate axes of FIGS. 1, 3A, and 3B), thereby defining a pocket 307B between the distal end 111-2 of the body 111 and the second extension piece 306B-2.

As depicted in FIGS. 1, 3A, and 3B, the first coupling member 306A and the second coupling member 306B are located opposite one another, radially outward of the circular protrusion 302 and the semi-circular channel 304. However, this is merely illustrative, and other locations and spacing are contemplated and included within the scope of the present disclosure. Further, while the present aspect includes a pair of coupling members (e.g., the first coupling member 306A and the second coupling member 306B), this is also merely illustrative. That is, other amounts of coupling members are also contemplated and included within the scope of the present disclosure.

Turning now to FIGS. 1 and 4A, the dual chamber mixing syringe 120 generally includes a body 121 having a distal end 121-1 and a proximal end 121-2 spaced a distance apart from the distal end 121-1. The body 121 also defines a pair of syringes 122.

As described above, the first pair of syringes 112 is configured having the first component chamber 112A, the second component chamber 112B, and the actuator 113 having the first piston 114A and the second piston 114B. However, initially (e.g., as a deliverable from the manufacturer), the first component chamber 112A does not yet contain the prepared first sealant component and the second component chamber 112B does not yet contain the prepared second sealant component. Rather, initially, the first component chamber 112A initially contains a powder or solution component of the first sealant component of the multi-component sealant, and the second component chamber 112B initially contains a powder or solution component of the second sealant component of the multi-component sealant. As used herein, each solution forming component is a solute and solvent combination, and may include, for example, a suspension or hydrogel.

The second pair of syringes 122 includes an actuator 123, a first mixing chamber 122A having a first mixing port 122A-1, and a second mixing chamber 122B having a second mixing port 122B-1. In some aspects, the first mixing chamber 122A and the second mixing chamber 122B are arranged in a substantially longitudinally parallel arrangement.

The actuator 123 includes a third piston 124A, a fourth piston 124B, and a handle 125. The handle 125 is in the form of a link member that perpendicularly extends between, and is connected to, each of third piston 124A and fourth piston 124B to facilitate simultaneous movement of the third piston 124A and the fourth piston 124B with the depression or retraction of the handle 125. The third piston 124A is in the form of a plunger that is positioned in the first mixing chamber 122A and the fourth piston 124B is in the form of a plunger that is positioned in the second mixing chamber 122B. The first mixing chamber 122A initially contains a fluid component of the first sealant component of the multi-component sealant, and the second mixing chamber 122B initially contains a fluid component of the second sealant component of the multi-component sealant. As used herein, each of the fluid component within the first mixing chamber 122A and/or the fluid component within the second mixing chamber 122B may be, or include, water or some other liquid.

The first mixing port 122A-1 of the first mixing chamber 122A and the second mixing port 122B-1 of the second mixing chamber 122B may be arranged within a proximal end 121-2 of the body 121 of the dual chamber mixing syringe 120. The first mixing port 122A-1 and the second mixing port 122B-1 are generally fluid outputs that are aligned with other ports of other components as described herein such that the respective fluid components initially within the mixing chambers 122A, 122B can be dispensed from and/or received (with the powder or solution initially within the component chambers 112A, 112B) within the respective mixing chambers 122A, 122B. In some aspects, the first mixing port 122A-1 may be concentrically aligned with the first mixing chamber 122A and the second mixing port 122B-1 may be concentrically aligned with the second mixing chamber 122B. However, in other aspects, such as the aspect depicted in FIG. 1, the first mixing port 122A-1 may be located radially inward of a central area of the first mixing chamber 122A and the second mixing port 122B-1 may be located radially inward of a central area of the second mixing chamber 122B such that the first mixing port 122A-1 and the second mixing port 122B-1 are as close as possible to a center axis C2 of the body 121 of the dual chamber mixing syringe 120 to facilitate alignment with the other components of the sealant delivery system 100 described herein.

Still referring to FIGS. 1 and 4A, the corresponding quarter turn connector 400 of the dual chamber mixing syringe 120 includes a circular recess 402 sized to receive the circular protrusion 302 on the distal end 111-2 of the sealant applicator 110 and a ramped lip 404 extending radially outward of the circular recess 402. In some aspects, the corresponding quarter turn connector 400 of the dual chamber mixing syringe 120 may further include a quarter circular extension (not shown) that extends proximally (e.g., in the +x direction of the coordinate axes of FIG. 1) from the ramped lip 404.

The circular recess 402 is generally a recess that is defined by a wall 403 extending proximally (e.g., in the +x direction of the coordinate axes of FIG. 1) from the proximal end 121-2 of the body 121 of the dual chamber mixing syringe 120. The wall 403 extends around the center axis C2 of the body 121 of the dual chamber mixing syringe 120 to form the circular recess 402. The wall 403 may be shaped and sized such that the circular recess 402 formed thereby corresponds to the shape and size of the circular protrusion 302 of the sealant applicator 110. In some aspects, the wall 403 may be an extension of side walls of the body 121 of the dual chamber mixing syringe 120.

The plurality of mixing ports 122A-1, 122B-1 extend out of the proximal end 121-2 of the body 121 of the dual chamber mixing syringe 120 within the circular recess 402. That is, the circular recess 402 includes the plurality of mixing ports 122A-1, 122B-1 therein. In some aspects, the circular recess 402 may include one or more features (e.g., additional recesses, retention pieces, channels, etc.) that are adapted to hold at least one seal (e.g., a sealing element or the like) around the plurality of mixing ports 122A-1, 122A-2. For example, a first seal 126A may be held within the circular recess 402 around the first mixing port 122A-1 and a second seal 126B may be held within the circular recess 402 around the second mixing port 122B-1. The seals 126A, 126B may each be any seal that allows the dual chamber mixing syringe 120 to form a seal with the sealant applicator 110 when brought together as described herein such that the first mixing port 122A-1 is joined and sealed with the first output port 112A-1 (e.g., to form a fluid coupling between the first mixing port 122A-1 and the first output port 112A-1) and the second mixing port 122B-1 is joined and sealed with the second output port 112B-1 (e.g., to form a fluid coupling between the second mixing port 122B-1 and the second output port 112B-1). For example, the seals may be O-rings, stadium shaped seals, oval seals, and/or the like. While a single seal is depicted herein for each port, the present disclosure is not limited to such. For example, a single seal, such as a figure eight shaped gasket or the like, may be used to individually seal the ports as described herein. However, it should be understood that the ports (e.g., the first mixing port 122A-1 and the second mixing port 122B-1) remain sealed from one another to avoid cross contamination of the components within the respective mixing chambers 122A, 122B. It should also be understood that the term "seal" is not meant to be limiting, and may encompass any type of sealing element, sealing device, or the like.

The ramped lip 404 is generally formed by the wall 403 that defines the circular recess 402. That is, the ramped lip 404 generally extends radially outward from the wall 403. The ramped lip 404 shaped and sized to be received and retained by the pair of coupling members 306A, 306B when the corresponding quarter turn connector 400 is rotated relative to the quarter turn connector 300, as described herein. That is, the ramped lip 404 has a first thickness (e.g., as defined along the x-axis of the coordinate axes of FIG. 1) at one or more first portions thereof, which gradually increases to a second thickness (e.g., as defined along the x-axis of the coordinate axes of FIG. 1) at one or more second portions thereof to allow for compressed holding of the ramped lip 404 by the pair of coupling members 306A, 306B within the pockets 307A, 307B thereof. In some aspects, the ramped lip may extend around an entire periphery of the wall 403. In other aspects, the ramped lip may extend only around a portion of the periphery of the wall 403. In such aspects, a plurality of ramped lips may be used.

While not depicted, in some aspects, the quarter circular extension may extend proximally from the ramped lip 404. The quarter circular extension shaped to be received within the semi-circular channel 304 (FIG. 3B) and slidable within the semi-circular channel 304 (FIG. 3B) in a quarter turn motion from an unlocked state to a locked state. The arc length of such a quarter circular extension may correspond to the length of the semi-circular channel 304 such that, when the quarter circular extension is inserted into the semi-circular channel 304 and turned, the respective lengths prevent more than a quarter turn rotation.

In some aspects, the corresponding quarter turn connector 400 further includes a pair of stops 406 (only one depicted in FIG. 4A). Each one of the pair of stops 406 extends distally (e.g., in the -x direction of the coordinate axes of FIG. 4A, toward the distal end 121-1 of the body 121) from the ramped lip 404. Each one of the pair of stops 406 is aligned on the corresponding quarter turn connector 400 such that the pair of stops 406 contact the coupling members 306A, 306B during a quarter turn rotating motion to hinder further rotational movement of the corresponding quarter turn connector 400 relative to the quarter turn connector 300 beyond a quarter rotation. It should be understood that any number of stops may be used, though the number of stops generally corresponds to the same number of coupling members (or less than the number of coupling members). For example, if the quarter turn connector 300 includes two coupling members 306A, 306B, the corresponding quarter turn connector 400 may include one stop 406 or two stops 406. In some aspects, the pair of stops 406 may extend radially outward from the wall 403 and may not be connected to the ramped lip 404.

Referring now to FIGS. 1 and 4B, the injection needle assembly 130 has a proximal end 130-1 that extends proximally (in the +x direction of the coordinate axes of FIG. 1) and a distal end 130-2 that extends distally (e.g., in the -x direction of the coordinate axes of FIG. 1). The injection needle assembly 130 generally includes a hub 131 and an elongate hollow stylet 132 that extends distally (e.g., in the -x direction of the coordinate axes of FIG. 1) from hub 131. The elongate hollow stylet 132 has a proximal end 132-1 and a distal end 132-2. The hub 131 is fixedly attached, e.g., through overmolding, adhesive and/or pressed fit, to the proximal end 132-1 of the elongate hollow stylet 132. The hub 131 includes a plurality of input ports (e.g., a first input port 132A-1 and a second input port 132B-1) of the injection needle assembly 130. The hub 131 is configured for removable connection to the sealant applicator 110 via the corresponding quarter turn connector 450 such that, when connected, the first input port 132A-1 of the injection needle assembly 130 is aligned and sealed with the first output port 112A-1 of the sealant applicator 110 and the second input port 132B-1 of the injection needle assembly 130 is aligned and sealed with the second output port 112B-1 of the sealant applicator 110, as described in greater detail herein.

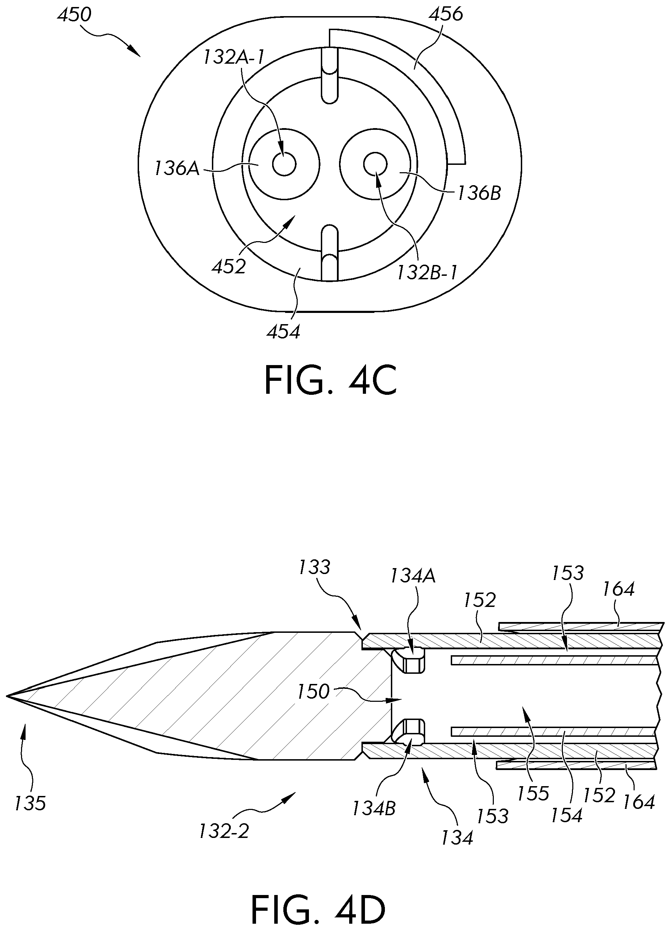

The elongate hollow stylet 132 of injection needle assembly 130 is configured to facilitate fluid communication with the plurality of output ports 112A-1, 112B-1 of the sealant applicator 110 so as to receive the two components of the multi-component sealant from sealant applicator 110 and direct the two components to the distal end 132-2 thereof for mixing and delivery. Referring briefly to FIG. 4D, the distal end 132-2 has a closed distal end 133 and a plurality of side ports 134 (e.g., two, three, or more) proximal to the closed distal end 133. The closed distal end 133 of the elongate hollow stylet 132 may be, for example, a closed stylet needle tip 135.

Referring to FIGS. 1, 4B, and 4D, the elongate hollow stylet 132 may be constructed, for example, by an elongate cannula 150 being fixedly connected to the closed stylet needle tip 135, with the plurality of side ports 134 being located in the elongate cannula 150 in a distal chamber 151 thereof that is immediately proximal to the closed stylet needle tip 135. More particularly, the elongate cannula 150 of the elongate hollow stylet 132 defines an outer side wall 152 that surrounds an outer lumen 153 of the elongate hollow stylet 132. In addition, located within the outer lumen 153 of the elongate cannula 150 is an inner side wall 154 that surrounds an inner lumen 155, the inner lumen being concentric with the outer lumen such that a passageway is defined between the inner side wall 154 and the outer side wall 152. Furthermore, a distal opening 153-1 of the outer lumen 153 and a distal opening 155-1 of the inner lumen 155 are open to the distal chamber 151 of the elongate hollow stylet 132.

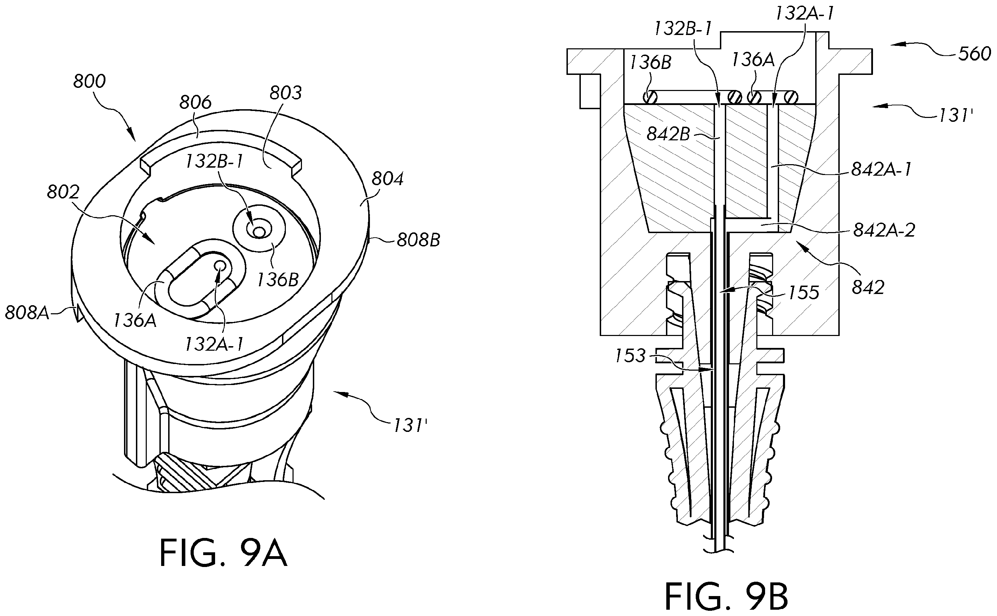

Referring to FIGS. 1 and 4D, the hub 131 may further include a plurality of passageways therein for fluidly coupling the first input port 132A-1 and the second input port 132B-1 to the distal chamber 151. For example, in some aspects, a first channel 142A may couple the first input port 132A-1 to the outer lumen 153 by extending through the hub 131 from the first input port 132A-1 to the outer lumen 153. In another example, a second channel 142B may couple the second input port 132B-1 to the inner lumen 155 by extending through the hub 131 from the second input port 132B-1 to the inner lumen 155. The positioning of the first channel 142A and the second channel 142B may be dependent on a location of the first input port 132A-1 and the second input port 132B-1. For example, in the aspect depicted in FIGS. 1 and 4B, the first input port 132A-1 and the second input port 132B-1 are spaced to be aligned with the output ports 112A-1, 112B-1 of the sealant applicator 110. As such, the first input port 132A-1 and the second input port 132B-1 are generally spaced the same distance radially outward from the center axis C3 of the injection needle assembly 130. In such an aspect, the first channel 142A may extend longitudinally toward the distal end 132-2 of the injection needle assembly 130 (e.g., parallel with the center axis C3 of the needle assembly) and couple to the outer lumen near a distal end of the hub 131. The second channel 142B may extend at an angle with respect to the center axis C3 from the second input port 132B-1 to the inner lumen 155. However, it should be understood that the present disclosure is not limited to such. For example, in another aspect, as depicted in FIGS. 9A-9B, an alternative first channel 842A may have a first channel portion 842A-1 that extends longitudinally through the alternative hub 131' and a second channel portion 842A-2 that extends laterally (e.g., transverse to the first channel portion 842A-1) to fluidly couple the first input port 132A-1 to the outer lumen 153. An alternative second input port 132B-1' may be located at the center of the alternative hub 131' and an alternative second channel 842B may extend longitudinally from the alternative second input port 132B-1' to the inner lumen 155.

Referring again to FIGS. 1 and 4D, the plurality of side ports 134 radially extend from the distal chamber 151 and through the outer side wall 152 of the elongate hollow stylet 132 at the distal end 132-2 thereof. The closed stylet needle tip 135 is defined, at least in part, by the closed distal end 133 of the elongate hollow stylet 132. The closed stylet needle tip 135 is attached (e.g., welded, press fit, or with adhesive) to the elongate cannula 150 to distally close the distal chamber 151 of the elongate cannula 150 of the elongate hollow stylet 132. That is, the closed stylet needle tip 135 terminates a distal extent of the distal chamber 151 of the elongate hollow stylet 132. When the delivery apparatus 250 (FIG. 2B) is assembled, the plurality of side ports 134 are in fluid communication with the plurality of output ports 112A-1, 112B-1 of the sealant applicator 110 by way of the plurality of input ports 132A-1, 132B-1, the outer lumen 153, the inner lumen 155, and the distal chamber 151 of the elongate hollow stylet 132.

In some aspects, as particularly shown in FIG. 4D, the plurality of side ports 134 in the distal end 132-2 of elongate hollow stylet 132 includes a first side port 134A, a second side port 134B, and a third side port (not shown), which are located in the closed distal end 133 and arranged, e.g., in a ring pattern, around a perimeter of elongate hollow stylet 132, such as, for example, in 120 degree increments. In one application, for example, the plurality of side ports 134 include at least three side ports (e.g., 3 to 7 side ports) to have the at least three side ports near, but proximal to, the closed stylet needle tip 135 such that the flowable multi-component sealant may be delivered 360 degrees around elongate hollow stylet 132, so as to seal around the perforation/close to the perforation created by the closed stylet needle tip 135. As another example, it is contemplated that in some applications it may be desirable to have the plurality of side ports 134 configured as two, or two pairs, of diametrically opposed side ports.

Optionally, it is further contemplated that the plurality of side ports 134 may include at least two longitudinally spaced side ports, such as for example, a side port longitudinally spaced (e.g. 1 to 3 millimeters) proximal to another side port. For example, the plurality of side ports 134 may include two rings of three side ports arranged around a perimeter of elongate hollow stylet 132, wherein the two rings of three side ports are longitudinally spaced in the distal end 132-2 of the elongate hollow stylet 132.

In an embodiment, these features of the injection needle assembly 130 (e.g., the elongate hollow stylet 132 including the inner lumen 155, the outer lumen 153, the distal chamber 151, and the plurality of side ports 134 may advantageously result in an assembly that separately delivers the separate components of the multi-component sealant to the point where the sealant is to be applied such that the components are not mixed together until they reach the point where the sealant is to be applied, thereby avoiding issues relating to clogging or the like.

The injection needle assembly 130 may be used in conjunction with the introducer cannula 160 (also sometimes referred to in the art as a coaxial introducer needle) to allow elongate hollow stylet 132 to be removed from the introducer cannula 160, while maintaining access to the procedure site with the coaxial introducer needle. That is, the delivery apparatus 250 may be removed from the introducer cannula 160 and replaced with a variety of other instruments, such as a biopsy device or another stylet. In some aspects, the introducer cannula 160 has a coaxial hub 162, a coaxial cannula 164, a cannula lumen 166 and a distal annular rim 168. The cannula lumen 166, for example, is configured (e.g., having a cylindrical shape) to receive the elongate hollow stylet 132 of the injection needle assembly 130. When the elongate hollow stylet 132 of the injection needle assembly 130 is fully inserted into the cannula lumen 166 of the introducer cannula 160 (e.g., distal movement of the elongate hollow stylet 132 is stopped by contact of the hub 131 of the injection needle assembly 130 with the coaxial hub 162 of the introducer cannula 160, the plurality of side ports 134 of the elongate hollow stylet 132 are located distal to the distal annular rim 168 of the introducer cannula 160. That is, the distal end 132-2 of the elongate hollow stylet 132 extends beyond the distal annular rim 168 such that the introducer cannula 160 does not block the plurality of side ports 134.

Referring to FIGS. 1 and 4B-4C, the corresponding quarter turn connector 450 of the injection needle assembly 130 includes a circular recess 452 sized to receive the circular protrusion 302 on the distal end 111-2 of the sealant applicator 110 and a ramped lip 454 extending radially outward of the circular recess 452. In some aspects, the corresponding quarter turn connector 450 of the injection needle assembly 130 may further include a quarter circular extension 456 (FIG. 4C) that extends proximally (e.g., in the +x direction of the coordinate axes of FIG. 1) from the ramped lip 454.

The circular recess 452 is generally a recess that is defined by a wall 453 extending proximally (e.g., in the +x direction of the coordinate axes of FIG. 1) from a proximal end 130-1 of the injection needle assembly 130 (e.g., extending proximally from the hub 131 of the injection needle assembly 130). The wall 453 extends around the center axis C3 of the injection needle assembly 130 to form the circular recess 452. The wall 453 may be shaped and sized such that the circular recess 452 formed thereby corresponds to the shape and size of the circular protrusion 302 of the sealant applicator 110. In some aspects, the wall 453 may be an extension of side walls of the hub 131 of the injection needle assembly 130.

The plurality of input ports 132A-1, 132B-1 extend out of the proximal end 130-1 of the injection needle assembly 130 within the circular recess 452. That is, the circular recess 452 includes the plurality of input ports 132A-1, 132B-1 therein. In some aspects, the circular recess 452 may include one or more features (e.g., additional recesses, retention pieces, channels, etc.) that are adapted to hold at least one seal around the plurality of input ports 132A-1, 132B-1. For example, a first seal 136A may be held within the circular recess 452 around the first input port 132A-1 and a second seal 136B may be held within the circular recess 452 around the second input port 132B-1. The seals 136A, 136B may each be any seal that allows the injection needle assembly 130 to form a seal with the sealant applicator 110 when brought together as described herein such that the first input port 132A-1 is joined and sealed with the first output port 112A-1 (e.g., to form a fluid coupling between the first input port 132A-1 and the first output port 112A-1) and the second input port 132B-1 is joined and sealed with the second output port 112B-1 (e.g., to form a fluid coupling between the second input port 132B-1 and the second output port 112B-1). For example, the seals may be O-rings, stadium shaped seals, oval seals, and/or the like. While a single seal is depicted herein for each port, the present disclosure is not limited to such. For example, a single seal, such as a figure eight shaped gasket or the like, may be used to individually seal the ports as described herein. However, it should be understood that the ports (e.g., the first input port 132A-1 and the second input port 132B-1) remain sealed from one another to avoid premature combining of components prior to reaching the distal chamber 151 (FIG. 4D).

Still referring to FIGS. 1, 4B, and 4C, the ramped lip 454 is generally formed by the wall 453 that defines the circular recess 452. That is, the ramped lip 454 generally extends radially outward from the wall 453. The ramped lip 454 is shaped and sized to be received and retained by the pair of coupling members 306A, 306B when the corresponding quarter turn connector 450 is rotated relative to the quarter turn connector 300, as described herein. That is, the ramped lip 454 has a first thickness (e.g., as defined along the x-axis of the coordinate axes of FIG. 1) at one or more first portions thereof, which gradually increases to a second thickness (e.g., as defined along the x-axis of the coordinate axes of FIG. 1) at one or more second portions thereof to allow for compressed holding of the ramped lip 454 by the pair of coupling members 306A, 306B within the pockets 307A, 307B thereof. In some aspects, the ramped lip 454 may extend around an entire periphery of the wall 453. In other aspects, the ramped lip may extend only around a portion of the periphery of the wall 453. In such aspects, a plurality of ramped lips may be used.

In some aspects, the quarter circular extension 456 may extend proximally from the ramped lip 454. The quarter circular extension 456 is shaped to be received within the semi-circular channel 304 (FIG. 3B) and slidable within the semi-circular channel 304 (FIG. 3B) in a quarter turn motion from an unlocked state to a locked state. The arc length of such a quarter circular extension 456 may correspond to the length of the semi-circular channel 304 such that, when the quarter circular extension 456 is inserted into the semi-circular channel 304 and turned, the respective lengths prevent more than a quarter turn rotation.

In some aspects, the corresponding quarter turn connector 450 further includes a pair of stops 458 (only one depicted in FIG. 4B). Each one of the pair of stops 458 extends distally (e.g., in the -x direction of the coordinate axes of FIG. 4B, toward the distal end 132-2 of the injection needle assembly 130) from the ramped lip 454. Each one of the pair of stops 458 is aligned on the corresponding quarter turn connector 450 such that the pair of stops 456 contact the coupling members 306A, 306B during a quarter turn rotating motion to hinder further rotational movement of the corresponding quarter turn connector 450 relative to the quarter turn connector 300 beyond a quarter rotation. It should be understood that any number of stops may be used, though the number of stops generally corresponds to the same number of coupling members (or less than the number of coupling members). For example, if the quarter turn connector 300 includes two coupling members 306A, 306B, the corresponding quarter turn connector 450 may include one stop 456 or two stops 456. In some aspects, the pair of stops 456 may extend radially outward from the wall 453 and may not be connected to the ramped lip 454.

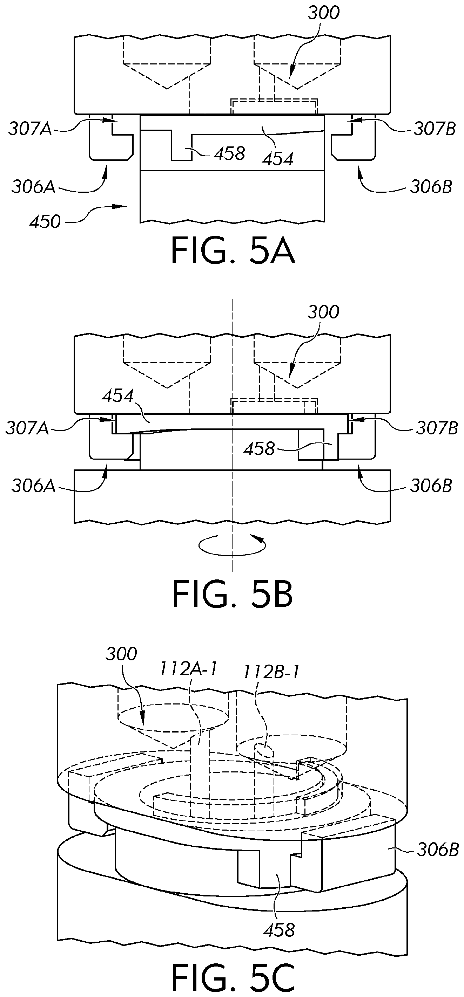

In an embodiment, the features of the quarter turn connectors 300, 400, 450 may advantageously result in a structure that allows a user to quickly connect and subsequently disconnect components (e.g., the sealant applicator 110 with the dual chamber mixing syringe 120 and the needle assembly 130) in such a manner that a user can easily confirm that components are correctly sealed and aligned with one another to ensure the correct materials held within are mixed together and then subsequently delivered to a site on a subject. This improvement is shown in the quarter turn operation of the various components of the sealant delivery system 100 (FIG. 1) is depicted in FIGS. 5A-5C. While FIGS. 5A-5C depict a coupling of the corresponding quarter turn connector 450 of the injection needle assembly 130 (FIG. 1), it should be understood that the quarter turn connector 400 of the dual chamber mixing syringe 120 may function in a similar manner. As shown in FIG. 5A, the corresponding quarter turn connector 450 and the quarter turn connector 300 are brought together. Initially, the pair of coupling members 306A, 306B are not engaged with the ramped lip 454 (e.g., the ramped lip 454 is not received within the pockets 307A, 307B. Referring to FIG. 5B, as the corresponding quarter turn connector 450 is rotated relative to the quarter turn connector 300 (e.g., by rotating one or both of the corresponding quarter turn connector 450 and the quarter turn connector 300), the ramped lip 454 begins to engage with the coupling members 306A, 306B by sliding into the pockets 307A, 307B thereof. This motion continues until the stops 458 prevent further rotational movement due to contact with the coupling members 306A, 306B (e.g., as shown in FIGS. 5B and 5C), at which point the output ports 112A-1, 112B-1 are appropriately aligned and sealed with the input ports (not shown). It should be appreciated that, given the components described herein, the corresponding quarter turn connector 450 and the quarter turn connector 300 can only be aligned in one particular manner with respect to each other, thereby avoiding issues related to misalignment or incorrect alignment.

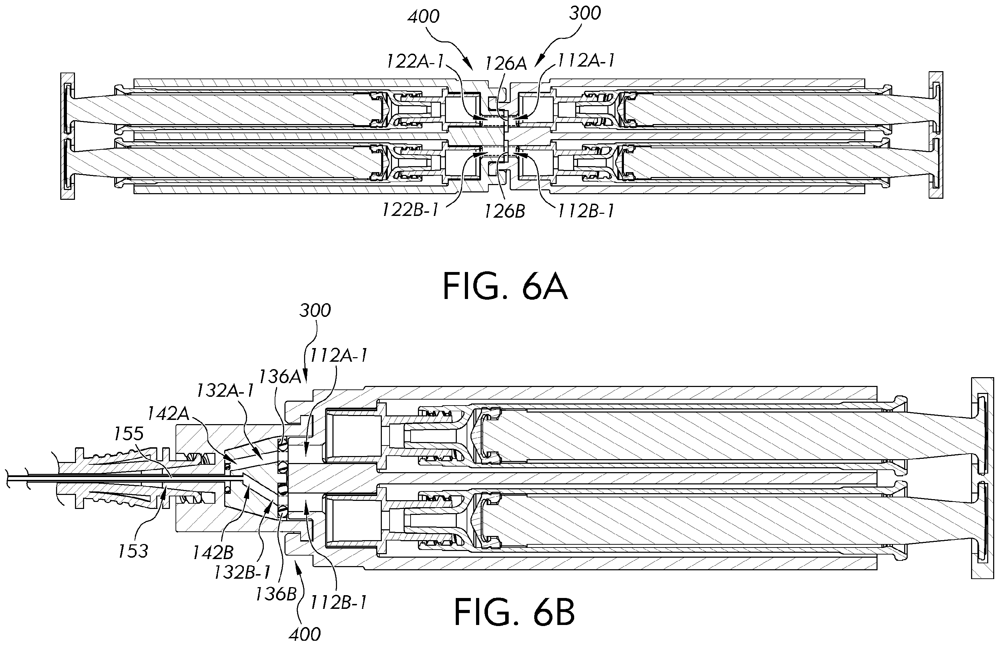

As a result of the quarter turn operation depicted in FIGS. 5A-5C, the various ports are appropriately arranged and sealed with respect to one another to ensure that materials can pass through without leakage. For example, as depicted in FIG. 6A, as a result of coupling via the respective quarter turn connectors 300, 400, the first output port 112A-1 is aligned and sealed with the first mixing port 122A-1 using the seal 126A therebetween. In addition, the second output port 112B-1 is aligned and sealed with the second mixing port 122B-1 using the seal 126B therebetween. In another example, as depicted in FIG. 6B, as a result of coupling via the respective quarter turn connectors 300, 450, the first output port 112A-1 is aligned and sealed with the first input port 132A-1 using the seal 136A therebetween. In addition, the second output port 112B-1 is aligned and sealed with the second input port 132B-1 using the seal 136B therebetween. As a result, a fluid connection is achieved between the first output port 112A-1 and the outer lumen 153 via the first input port 132A-1 and the first channel 142A. Similarly, a fluid connection is achieved between the second output port 112B-1 and the inner lumen 155 via the second input port 132B-1 and the second channel 142B.

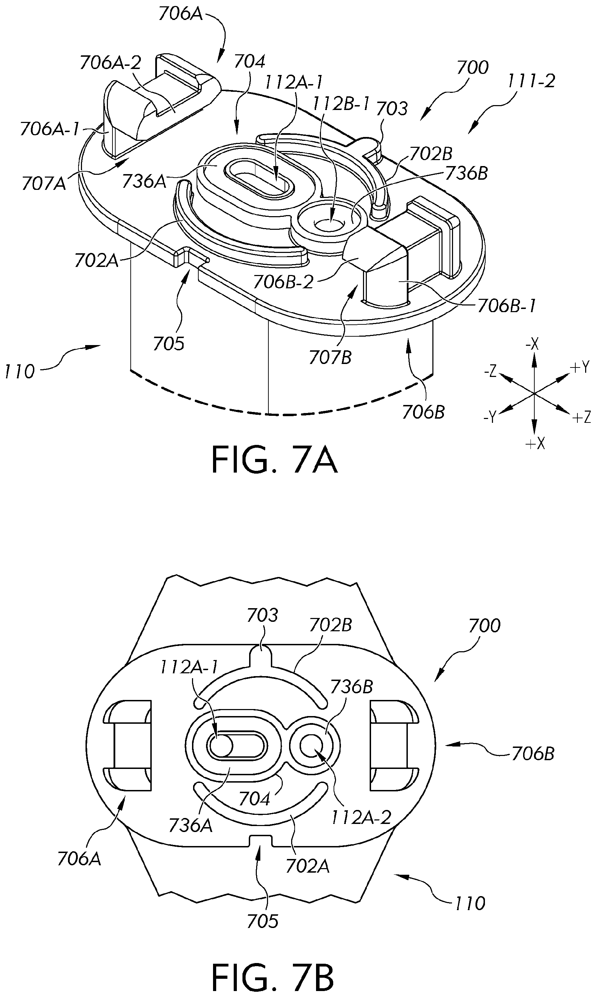

It should be understood that the various quarter turn connectors described herein with respect to FIGS. 1, 2A-2B, 3A-3B, 4A-4D, 5A-5C, and 6A-6B are merely illustrative and other quarter turn connectors are also contemplated and included within the scope of the present disclosure. For example, other illustrative quarter turn connectors are depicted in FIGS. 7A-7E.

Referring now to FIGS. 7A-7B, another illustrative quarter turn connector 700 disposed on the sealant applicator 110 is depicted. As shown in FIGS. 7A-7B, the quarter turn connector 700 includes a first arc shaped protrusion 702A, a second arc shaped protrusion 702B, one or more seal containment protrusions 704 and/or a pair of coupling members 706A, 706B.

The one or more seal containment protrusions 704 extend a distance distally (e.g., in the -x direction of the coordinate axes depicted in FIG. 7A) from the distal end 111-2 of the sealant applicator 110. The one or more seal containment protrusions 704 are generally disposed around the output ports 112A-1, 112B-1 and are shaped and sized to retain seals 736A, 736B therein such that the seals 736A, 736B surround the output ports 112A-1, 112B-1. Accordingly, the one or more seal containment protrusions 704 may generally be shaped and sized to correspond to the shape and size of the seals 736A, 736B such that the seals 736A, 736B can be press fit within the one or more seal containment protrusions 704. In some aspects, the one or more seal containment protrusions 704 may be a single structure that is shaped and sized to hold the seals 736A, 736B, as generally depicted in FIGS. 7A and 7B. In other aspects, the one or more seal containment protrusions 704 may be separate structures disposed around each of the output ports 112A-1, 112A-B to retain separate seals 736A, 736B therein. In some embodiments, the one or more seal containment protrusions 704 may be omitted. Rather, the seals 736A, 736B may be disposed within recesses, channels, or the like or may be fixedly attached to the distal end 111-2 of the sealant applicator 110 (e.g., via an adhesive, an attachment device, or the like). The seals 736A, 736B may be similar to the seals 136A, 136B (FIG. 1) as previously discussed herein.

The first arc shaped protrusion 702A extends a distance distally (e.g., in the -x direction of the coordinate axes depicted in FIG. 7A) from the distal end 111-2 of the sealant applicator 110. In addition, the first arc shaped protrusion 702A is generally disposed radially outward from the one or more seal containment protrusions 704, the seals 736A. 736B, and the output ports 112A-1, 112B-1. The distance that the first arc shaped protrusion 702A extends from the distal end 111-2 of the sealant applicator 110 is generally the same or slightly less than that of the distance that the one or more seal containment protrusions 704 and/or the seals 736A, 736B such that, when the sealant applicator 110 is joined together with one of the other components described herein, the seals 736A, 736B are compressed between components to an extent that avoids leakage. However, it should be understood that the distance may also be longer in embodiments where the second arc shaped protrusion 702B is fit within a channel of a corresponding quarter turn connector. The first arc shaped protrusion 702A is generally shaped and sized to fit within a corresponding recess and rotate within the corresponding recess, as described in greater detail herein.

The second arc shaped protrusion 702B extends a distance distally (e.g., in the -x direction of the coordinate axes depicted in FIG. 7A) from the distal end 111-2 of the sealant applicator 110. In addition, the second arc shaped protrusion 702B is generally disposed radially outward from the one or more seal containment protrusions 704, the seals 736A. 736B, and the output ports 112A-1, 112B-1. In some aspects, the distance that the second arc shaped protrusion 702B extends from the distal end 111-2 of the sealant applicator 110 is generally the same or slightly less than that of the distance that the one or more seal containment protrusions 704 and/or the seals 736A, 736B such that, when the sealant applicator 110 is joined together with one of the other components described herein, the seals 736A, 736B are compressed between components to an extent that avoids leakage. However, it should be understood that the distance may also be longer in embodiments where the second arc shaped protrusion 702B is fit within a channel of a corresponding quarter turn connector. The second arc shaped protrusion 702B is generally shaped and sized to fit within a corresponding recess and rotate within the corresponding recess, as described in greater detail herein.

In some aspects, the second arc shaped protrusion 702B may include a tab 703 extending radially outward therefrom. That is, the tab 703 extends from the second arc shaped protrusion 702B in a radially outward direction. The tab 703 may generally be any shape and size, particularly a shape and size that can slide within a channel of a corresponding quarter turn connector and move from one end of the channel to another end of the channel, as described herein. In some aspects, the tab 703 may be particularly located to ensure appropriate alignment of the quarter turn connector 700 with a corresponding quarter turn connector, as described herein. It should be appreciated that the tab 703 may be omitted in some aspects.

The first coupling member 706A and the second coupling member 706B each extend from the distal end 111-2 of the sealant applicator 110 and are generally shaped and sized to retain the dual chamber mixing syringe 120 (FIG. 1) or the injection needle assembly 130 (FIG. 1) when coupled to the sealant applicator 110. Still referring to FIGS. 7A-7B, each of the first coupling member 706A and the second coupling member 706B may be a bayonet style coupling member, an L-beam coupling member, or the like. For example, as particularly depicted in FIG. 7A, the first coupling member 706A extends distally at a particular distance from the distal end 111-2, turns about 90 degrees, and extends inward toward the center of the sealant applicator 110, resulting in a first extension piece 706A-1 that extends in a direction generally coplanar with the center of the sealant applicator 110 (e.g., along the x-axis of the coordinate axes of FIG. 7A) and a second extension piece 706A-2 that extends in a direction that is generally perpendicular to the center of the sealant applicator 110 (e.g., along the z-axis of the coordinate axes of FIG. 7A), thereby defining a pocket 707A between the distal end 111-2 and the second extension piece 706A-2. Similarly, the second coupling member 706B extends distally at a particular distance from the distal end 111-2 of the sealant applicator 110, turns about 90 degrees, and extends inward toward the center of the sealant applicator 110, resulting in a first extension piece 706B-1 that extends in a direction generally coplanar with the center of the sealant applicator 110 (e.g., along the x-axis of the coordinate axes of FIG. 7A) and a second extension piece 706B-2 that extends in a direction that is generally perpendicular to the center of the sealant applicator 110 (e.g., along the z-axis of the coordinate axes of FIG. 7A), thereby defining a pocket 707B between the distal end 111-2 of the sealant applicator 110 and the second extension piece 706B-2.

As depicted in FIGS. 7A-7B, the first coupling member 706A and the second coupling member 706B are located opposite one another, radially outward of the one or more seal containment protrusions 704, the seals 736A. 736B, the output ports 112A-1, 112B-1, and/or the first and second arc shaped protrusions 702A, 702B. However, this is merely illustrative, and other locations and spacing are contemplated and included within the scope of the present disclosure. Further, while the present aspect includes a pair of coupling members (e.g., the first coupling member 706A and the second coupling member 706B), this is also merely illustrative. That is, other amounts of coupling members are also contemplated and included within the scope of the present disclosure. In some embodiments, the first coupling member 706A and the second coupling member 706B are aligned to in a direction that is transverse to the direction in which the tab 703 extends. For example, as shown in FIG. 7A, the tab 703 extends outwardly in the +y direction of the coordinate axes of FIG. 7A, whereas the first coupling member 706A and the second coupling member 706B are aligned with each other along the +z/-z directions of the coordinate axes of FIG. 7A. Such an alignment may provide, for example, appropriate alignment of components and a quarter turn rotation, as described in greater detail herein.

FIGS. 7A-7B also depict a notch 705 disposed on a side of the distal end 111-2 of the sealant applicator 110. The notch 705 is generally provided as a visual indicator for verifying alignment of the quarter turn connector 700 with a corresponding quarter turn coupling member when joined. As such, the notch 705 may be particularly shaped, sized, and positioned to provide such a visual indicator. It should be understood that a notch is merely one illustrative example of a visual indicator that may be used. For example, other visual indicators such as indicia (numbers, words, arrows, and/or the like) that are embossed, formed, printed, attached (e.g., a sticker or the like) on the sealant applicator 110 may also be used.

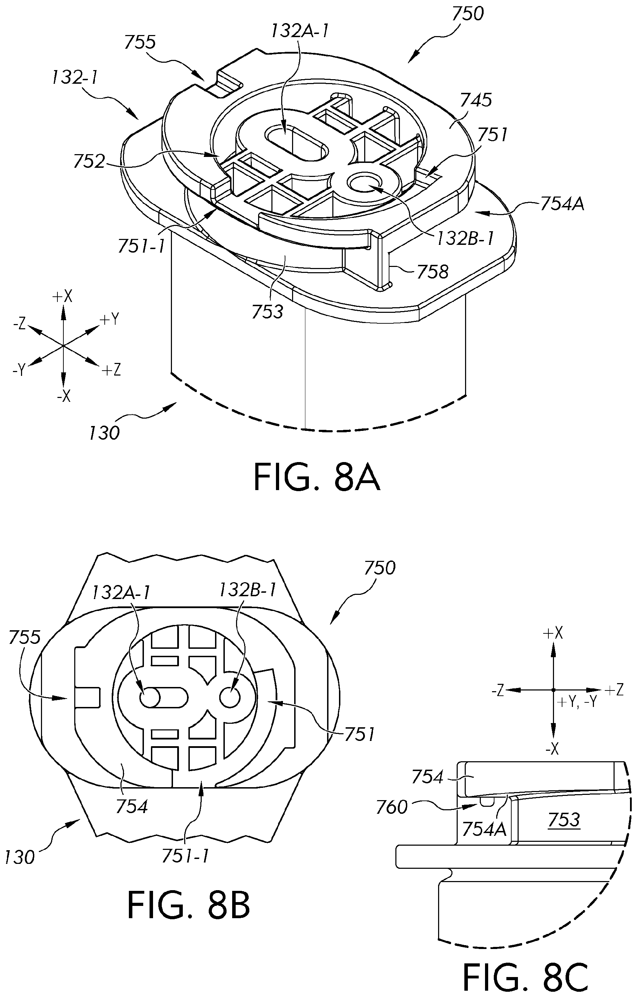

Turning now to FIGS. 8A-8C, the corresponding quarter turn connector 750 located on the injection needle assembly is depicted. While FIGS. 8A-8C specifically show the quarter turn connector 750 of the injection needle assembly 130, it should be understood that the dual chamber mixing syringe 120 (FIG. 1) may also be similarly structured with the components depicted in FIGS. 8A-8C.

As shown in FIGS. 8A-8B, the corresponding quarter turn connector 750 of the injection needle assembly 130 includes a circular recess 752 sized to receive the first and second arc shaped protrusions 702A, 702B on the distal end 111-2 of the sealant applicator 110 (FIGS. 7A-7B) and a ramped lip 754 extending radially outward of the circular recess 752.

The circular recess 752 is generally a recess that is defined by a wall 753 extending proximally (e.g., in the +x direction of the coordinate axes of FIG. 8A) from the proximal end 130-1 of the injection needle assembly 130 (e.g., extending proximally from the hub 131 of the injection needle assembly 130). The wall 753 extends around the center axis C3 (FIG. 1) of the injection needle assembly 130 to form the circular recess 752. The wall 753 may be shaped and sized such that the circular recess 752 formed thereby corresponds to the shape and size of the first and second arc shaped protrusions 702A, 702B of the sealant applicator 110. In some aspects, the wall 753 may be an extension of side walls of the hub 131 of the injection needle assembly 130.

The plurality of input ports 132A-1, 132B-1 extend out of the proximal end 130-1 of the injection needle assembly 130 within the circular recess 752. That is, the circular recess 752 includes the plurality of input ports 132A-1, 132B-1 therein. In some aspects, the circular recess 752 may include one or more features around the plurality of input ports 132A-1, 132B-1. For example, a platform within the circular recess 752 may extend out of the proximal end 130-1 of the injection needle assembly 130 around the input ports 132A-1, 132B-1 such that a channel is disposed within the circular recess 752 surrounding at least a portion of the input ports 132A-1. 132B-1. Such a platform may provide a contact surface upon which the seals 736A, 736B (FIGS. 7A-7B) abut when the injection needle assembly 130 is joined with the sealant applicator 110 as described herein.

Still referring to FIGS. 8A-8B, the ramped lip 754 is generally formed by the wall 753 that defines the circular recess 752. That is, the ramped lip 754 generally extends radially outward from the wall 753. Referring also to FIGS. 7A-7B, the ramped lip 754 is shaped and sized to be received and retained by the pair of coupling members 706A, 706B when the corresponding quarter turn connector 750 is rotated relative to the quarter turn connector 700, as described herein. That is, the ramped lip 754 has a first thickness (e.g., as defined along the x-axis of the coordinate axes of FIG. 8A) at one or more first portions thereof, which gradually increases to a second thickness (e.g., as defined along the x-axis of the coordinate axes of FIG. 8A) at one or more second portions thereof to allow for compressed holding of the ramped lip 754 by the pair of coupling members 706A, 706B within the pockets 707A, 707B thereof. In some aspects, the ramped lip 754 may extend around an entire periphery of the wall 753. In other aspects, the ramped lip may extend only around a portion of the periphery of the wall 753. In such aspects, a plurality of ramped lips may be used.

In some aspects, the corresponding quarter turn connector 750 may further include an arc shaped channel 751 disposed along a periphery of the circular recess 752. That is, the arc shaped channel 751 may be disposed radially outward of the circular recess 752 and radially inward of the ramped lip 754. In some aspects, the arc shaped channel 751 may be an extension of the circular recess 752, may have the same depth as the circular recess 752, and/or may not be divided from the circular recess 752. In other aspects, the arc shaped channel 751 may have a different depth than a depth of the circular recess 752 (e.g., may be shallower or deeper than the depth of the circular recess 752). Referring to FIGS. 7A-7B and 8A-8B, the arc shaped channel 751 is otherwise generally shaped and sized to receive the tab 703 extending from the second arc shaped protrusion 702B. That is, the arc shaped channel 751 may be shaped to receive the tab 703 when the injection needle assembly 130 is joined with the sealant applicator 110 and further may be shaped such that, when the injection needle assembly 130 and/or the sealant applicator 110 are rotated with respect to one another, the tab 703 rotates within the arc shaped channel 751. In some aspects, the arc shaped channel 751 may have an opening 751-1 on one end thereof to provide a visual indicator of the alignment of the tab prior to the rotating motion.

Referring to FIG. 8A, in some aspects, the corresponding quarter turn connector 750 further includes a pair of stops 758 (only one depicted in FIG. 8A). Each one of the pair of stops 758 extends distally (e.g., in the -x direction of the coordinate axes of FIG. 8A, toward the distal end 132-2 (FIG. 1) of the injection needle assembly 130) from the ramped lip 754. Referring also to FIGS. 7A-7B, each one of the pair of stops 758 is aligned on the corresponding quarter turn connector 750 such that the pair of stops 758 contact the coupling members 706A, 706B during a quarter turn rotating motion to hinder further rotational movement of the corresponding quarter turn connector 750 relative to the quarter turn connector 700 beyond a quarter rotation. It should be understood that any number of stops may be used, though the number of stops generally corresponds to the same number of coupling members (or less than the number of coupling members). For example, if the quarter turn connector 700 includes two coupling members 706A, 706B, the corresponding quarter turn connector 750 may include one stop 756 or two stops 756. In some aspects, the pair of stops 756 may extend radially outward from the wall 753 and may not be connected to the ramped lip 754.

In some aspects, the corresponding quarter turn connector 750 may further include a notch 755 in a portion of the ramped lip 754. The notch 755 is generally provided as a visual indicator for verifying alignment of the quarter turn connector 700 (FIGS. 7A-7B) with the corresponding quarter turn connector 750 when joined. For example, the notch 755 may align with the notch 705 (FIGS. 7A-7B) when the components are joined (e.g., after rotation to interlock the components). As such, the notch 755 may be particularly shaped, sized, and positioned to provide such a visual indicator. It should be understood that a notch is merely one illustrative example of a visual indicator that may be used. For example, other visual indicators such as indicia (numbers, words, arrows, and/or the like) that are embossed, formed, printed, attached (e.g., a sticker or the like) on the sealant applicator 110 may also be used.

Referring to FIG. 8C, in some embodiments, the corresponding quarter turn connector 750 further includes an extension piece 760 extending distally (e.g., in the -x direction of the coordinate axes of FIG. 8C) from a distally facing portion 754A (e.g., in the - x direction of the coordinate axes of FIG. 8C) of the ramped lip 754. Referring also to FIGS. 7A-7B, the extension piece 760 is generally shaped, sized, and/or configured to contact one of the coupling members 706A, 706B (e.g., the second extension piece 706A-2 of the first coupling member 706A or the second extension piece 706B-2 of the second coupling member) when the injection needle assembly 130 is joined with the sealant applicator 110 and during a rotational movement of the corresponding quarter turn connector 750 relative to the quarter turn connector 700 and cause the coupling member 706A, 706B to flex radially outward and then snap back into place when the rotational movement is completed, providing a haptic and audible feedback mechanism that the corresponding quarter turn connector 750 and the quarter turn connector 700 are appropriately coupled. That is, the extension piece 760 is shaped and located relative to the various other components described herein so that the extension piece clears (e.g., moves out of contact with) the coupling member 706A, 706B at the point where the quarter turn movement has been completed, thereby causing the snapping back of the coupling member 706A, 706B. In an embodiment, use of this feature (e.g., the extension piece 760) may advantageously result in feedback being provided to the user such that the user is provided with extra confirmation that the components are correctly connected and aligned via the quarter turn connectors.

Referring again to FIGS. 9A-9B, the other illustrative hub 131' is depicted. The hub 131', similar to the hub depicted in FIGS. 1, 4B, and 4C, includes a corresponding quarter turn connector 800 that includes a circular recess 802 defined by a wall 803, a ramped lip 804, a pair of stops 808A, 808B, and quarter circular protrusion 806. Such components are similar to the components previously described herein and are not discussed further herein for the purposes of brevity. However, as previously noted, the second input port 132B-1' is centrally located in the alternative hub 131'. Since this location is not aligned with the second output port 112B-1 when the components are mated as described herein, a stadium shaped gasket or the like as depicted in FIGS 9A-9B may provide a sealed fluid connection between the second output port 112B-1 with the second input port 132B-1' such that fluid can flow therebetween.

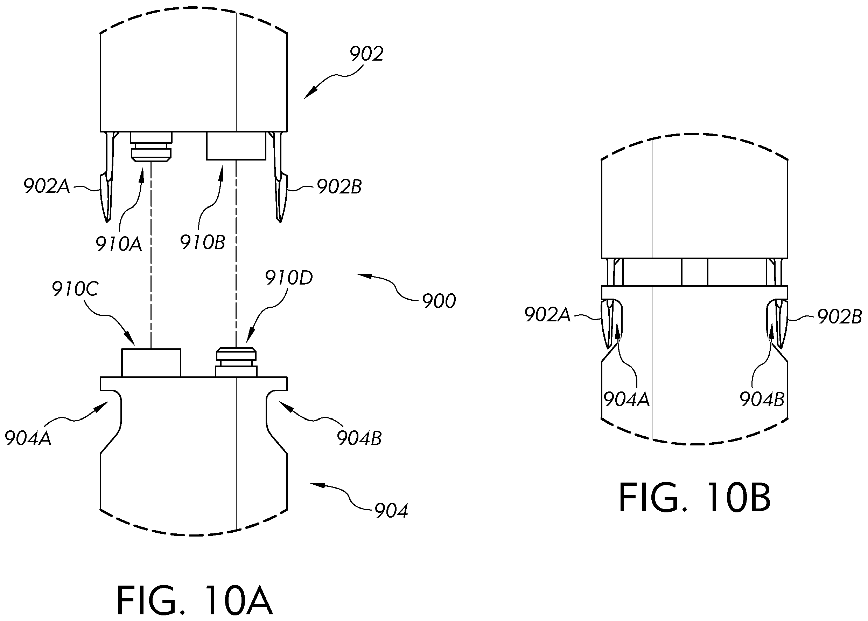

While the present disclosure relates primarily to the quarter turn coupling described herein, other components that can be used to quickly provide and remove a fluid connection between the various components of the sealant delivery system 100 (FIG. 1) described herein. One such illustrative example is depicted in FIGS. 10A-10B. As shown in FIG. 10A and with reference to FIG. 1, a clip fit connection 900 includes a male clip portion 902 (e.g., containing a first prong 902A and a second prong 902B) on one component (e.g., the sealant applicator 110 or the dual chamber mixing syringe 120/injection needle assembly 130) and a female receptacle portion 904 (e.g., containing a first receptacle 904A and a second receptacle 904B on another component (e.g., the other of the sealant applicator 110 or the dual chamber mixing syringe 120/injection needle assembly 130). As can be appreciated, the female receptacle portion 904 is shaped and sized to receive and restrain the male clip portion 902 therein to retain the two components together, as depicted in FIG. 10B. The connection is reversible by engaging a release mechanism disposed on one or more of the male clip portion 902 and/or the female receptacle portion 904. For example, in the aspect depicted in FIG. 10B, the first prong 902A and the second prong 902B of the male clip portion 902 can be squeezed toward one another to release a retention mechanism such that the male clip portion 902 can be slid out of the female receptacle portion 904. In addition, the components can be keyed so as to ensure appropriate alignment, as described herein. For example, as depicted in FIG. 10A and with reference to FIG. 1, a first male port 910A and a first female port 910B may be disposed on one component (e.g., the sealant applicator 110 or the dual chamber mixing syringe 120/injection needle assembly 130). Similarly, a second female port 910c and a second male port 910D may be disposed on another component (e.g., the other of the sealant applicator 110 or the dual chamber mixing syringe 120/injection needle assembly 130). As such, when the two components are joined together, the first male port 910A joins with the second female port 910C and the first female port 910B joins with the second male port 910D to fluidly couple components together, as described in greater detail herein.

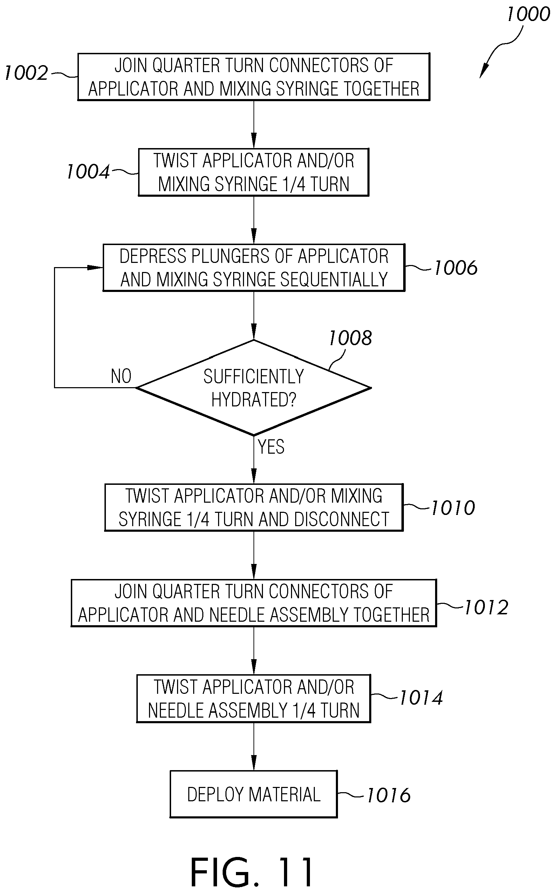

FIG. 11 depicts a flow diagram of an illustrative method 1000 of using the various components of the sealant delivery system 100 (FIG. 1) as described herein. While the method 1000 of FIG. 11 generally relates to use of the quarter turn connectors described herein, it should be understood that a similar process may be used for other connectors (e.g., the connectors described herein with respect to FIGS. 10A-10B), albeit without the rotating steps.

Referring to FIG. 11, the method 1000 includes joining the quarter turn connectors of the sealant applicator and the dual chamber mixing syringe together as described herein at block 1002. That is, the distal end of the sealant applicator (containing the quarter turn connector thereon) is brought together and placed in an initial alignment with the proximal end of the dual chamber mixing syringe (containing the corresponding quarter turn connector thereon). Once brought together and aligned, the sealant applicator and/or the dual chamber mixing syringe are rotated with respect to one another in a ¼ turn at block 1004 to lock the components together. In some aspects, an indicator of a correct alignment can be checked to verify the connection, as described herein. In some aspects, a tactile or audible click may provide feedback of a correct joining and alignment. In an embodiment, this feature may advantageously result in additional feedback that is provided to the user to reassure the user that the components are correctly aligned and joined.

At block 1006, the actuators (e.g., plungers) of the applicator and the dual chamber mixing syringe, respectively, may be depressed sequentially to cause the four separate contents contained therein (e.g., contents in each of the two chambers of the sealant applicator and in each of the two chambers of the dual chamber mixing syringe) to be mixed into two mixtures. The sequential depressing of the actuators may be completed any number of times. Accordingly, at decision block 1008 a determination may be made as to whether the two mixtures are sufficiently hydrated. Such a determination should generally be understood. If the mixtures are not sufficiently hydrated, the process may repeat at block 1006. If the mixtures are sufficiently hydrated, the process may continue to block 1010. Before proceeding to block 1010, the two mixtures may be located within the two chambers of the sealant applicator.

At block 1010, the sealant applicator may be disconnected from the dual chamber mixing syringe by rotating the two components relative to one another in a quarter turn in a direction opposite as the initial quarter turn completed at block 1004. The dual chamber mixing syringe may be discarded after being disconnected.

At block 1012, the disconnected sealant applicator may be joined with the needle assembly as described herein. That is, the distal end of the sealant applicator (containing the quarter turn connector thereon) is brought together and placed in an initial alignment with the proximal end of the needle assembly (containing the corresponding quarter turn connector thereon). Once brought together and aligned, the sealant applicator and/or the needle assembly are rotated with respect to one another in a ¼ turn at block 1014 to lock the components together. In some aspects, an indicator of a correct alignment can be checked to verify the connection, as described herein. In some aspects, a tactile or audible click may provide feedback of a correct joining and alignment. In an embodiment, this feature may advantageously result in additional feedback that is provided to the user to reassure the user that the components are correctly aligned and joined.

At block 1016, the components within the two chambers of the sealant applicator may be deployed via the needle assembly such that the components travel down the inner and outer lumens thereof to the distal mixing chamber, where they mix and exit out of the plurality of side ports. It should be understood that deploying the material according to block 1016 may be completed at a site where sealant is desired (e.g., the pleura region of a subject or the like).

It should now be understood that the present disclosure relates to various sealant delivery systems that include components that deliver a multicomponent sealant along an access path, including the pleura region, to the lung prior to performing a lung procedure, such as a biopsy or the like. The sealant delivery systems described herein include a dual chamber sealant applicator, a dual chamber mixing syringe, and an injection needle assembly. The sealant delivery systems described herein are configured such that a user can quickly and easily couple the dual chamber sealant applicator with the dual chamber mixing syringe in a manner that correctly aligns the applicator and mixing syringe so that the components within each can be mixed together. Once mixed, the user can just as easily disconnect the applicator from the mixing syringe and quickly and easily attach the injection needle assembly thereto for the purposes of delivering the sealant to a subject. To achieve this, the dual chamber sealant applicator includes a connector such as a quarter turn connector that allows for the quick joining and releasing of the dual chamber mixing syringe and the injection needle assembly, which both have a corresponding quarter turn connector. In addition, because the sealant materials located in the dual chamber sealant applicator quickly form a sealant product when combined together, it is necessary to ensure the combination occurs at the side where the sealant product is to be distributed. As such, the needle assembly described herein includes an elongate hollow stylet having a mixing chamber at a distal end thereof, and utilizes an inner lumen disposed concentrically within an outer lumen to define separate passageways extending from the two chambers of the applicator to the mixing chamber at the distal end.

While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A sealant applicator (110), comprising:
two chambers (112A, 112B) separate from one another, each chamber comprising at least one output port (112A-1, 112B-1) on a distal end thereof; and
a quarter turn connector (300, 700) disposed on a distal end (111-2) of the sealant applicator adjacent to the at least one output port of each chamber, the quarter turn connector shaped to releasably interlock with a corresponding quarter turn connector (450) of an injection needle assembly (130) comprising a plurality of input ports (132A-1, 132B-1) or with a corresponding quarter turn connector (400) of a dual chamber mixing syringe (120) comprising a plurality of mixing ports (122A-1, 122B-1), wherein, when the injection needle assembly or the dual chamber mixing syringe is coupled to the sealant applicator via the quarter turn connector, the plurality of input ports or the plurality of mixing ports are aligned and sealed with the at least one output port of each chamber of the sealant applicator;
wherein the quarter turn connector comprises:
a circular protrusion (302) extending distally from the distal end of the sealant applicator, the circular protrusion comprising the at least one output port of each of the two chambers;
a semi-circular channel (304) disposed within the distal end of the sealant applicator along a periphery of the circular protrusion; and
a pair of bayonet coupling members (306A, 306B) disposed radially outward of the circular protrusion and the semi-circular channel.

2. A sealant delivery system (100), comprising a sealant applicator (110) according to Claim 1.

3. The sealant delivery system according to claim 2,
wherein the corresponding quarter turn connector (450) of the injection needle assembly and the corresponding quarter turn connector of the dual chamber mixing syringe each comprise:
a circular recess (402) sized to receive the circular protrusion on the distal end of the sealant applicator, the circular recess comprising the plurality of input ports or the plurality of mixing ports disposed therein;
a ramped lip (404) extending radially outward of the circular recess, the ramped lip shaped and sized to be received and retained by the pair of bayonet coupling members when the corresponding quarter turn connector is rotated relative to the quarter turn connector; and
a quarter circular extension (456) extending proximally from the lip, the quarter circular extension shaped to be received within the semi-circular channel and slidable within the semi-circular channel in a quarter turn motion from an unlocked state to a locked state;
optionally wherein the corresponding quarter turn connector of the injection needle assembly and the corresponding quarter turn connector of the dual chamber mixing syringe each further comprise a pair of stops (406) extending distally from the ramped lip and aligned such that the pair of stops contact the pair of bayonet coupling members to hinder rotational movement of the corresponding quarter turn connector relative to the quarter turn connector beyond a quarter rotation.

4. The sealant delivery system according to claim 3, wherein a seal (126A, 126B, 136A, 136B) is disposed within the circular recess around each one of the plurality of input ports or the plurality of mixing ports disposed therein;
optionally wherein the seal is an O-ring or a stadium shaped seal.

5. The sealant delivery system according to any one of claims 2-4, further comprising:
the injection needle assembly, the injection needle assembly comprising:
a hub (131) comprising the corresponding quarter turn connector and the plurality of input ports; and
an elongate hollow stylet (132) that extends distally from the hub, the elongate hollow stylet having a proximal portion at the hub and a distal portion spaced apart from the proximal portion.

6. The sealant delivery system according to claim 5, wherein the elongate hollow stylet comprises:
an outer side wall (152) extending from the proximal portion to the distal portion and defining an outer lumen (153) that is fluidly coupled to at least a first one of the plurality of input ports;
an inner side wall (154) extending from the proximal portion to the distal portion and defining an inner lumen (155) disposed within the outer lumen such that the inner lumen is concentric with the outer lumen and has a cross-sectional size that is smaller than the outer lumen, the inner lumen being fluidly coupled to at least a second one of the plurality of input ports; and
a mixing chamber (151) disposed at the distal portion of the elongate hollow stylet, the mixing chamber fluidly coupled to the outer lumen and the inner lumen and comprising at least one side port (134A, 134B).

7. The sealant delivery system according to any one of claims 2-6, further comprising:
the dual chamber mixing syringe, the dual chamber mixing syringe comprising:
a first mixing chamber (122A) having a first mixing port (122A-1) of the plurality of mixing ports, and
a second mixing chamber (122B) having a second mixing port (122B-1) of the plurality of mixing ports.

8. An injection needle assembly, comprising:
a hub (131) having a hub quarter turn connector (450) that is configured to releasably interlock with a corresponding quarter turn connector disposed on a sealant applicator;
a plurality of input ports (132A-1, 132B-1) disposed within the hub; and
an elongate hollow stylet (132) that extends distally from the hub, the elongate hollow stylet having a proximal portion at the hub and a distal portion spaced apart from the proximal portion, the elongate hollow stylet comprising:
an outer side wall (152) extending from the proximal portion to the distal portion and defining an outer lumen (153) that is fluidly coupled to at least a first one of the plurality of input ports,
an inner side wall (154) extending from the proximal portion to the distal portion and defining an inner lumen (155) disposed within the outer lumen such that the inner lumen is concentric with the outer lumen and has a cross-sectional size that is smaller than the outer lumen, the inner lumen being fluidly coupled to at least a second one of the plurality of input ports, and
a mixing chamber (151) disposed at the distal portion of the elongate hollow stylet, the mixing chamber fluidly coupled to the outer lumen and the inner lumen and comprising at least one side port,
wherein, when the hub quarter turn connector interlocks with the quarter turn connector disposed on a
distal end of the sealant applicator, the plurality of input ports are each aligned and sealed with a corresponding output port of the sealant applicator;
wherein the hub quarter turn connector comprises:
a circular recess (402) sized to receive a circular protrusion on the distal end of the sealant applicator, the circular recess comprising the plurality of input ports disposed therein;
a lip (402) extending radially outward of the circular recess, the lip shaped and sized to be received within a pair of bayonet coupling members disposed on the sealant applicator; and
a quarter circular boss (456) extending proximally from the lip, the quarter circular boss shaped to be received within a semi-circular channel of the sealant applicator and slidable within the semi-circular channel in a quarter turn motion from an unlocked state to a locked state.

9. The injection needle assembly according to claim 8,
wherein the hub quarter turn connector further comprises a pair of stops (406) extending distally from the lip and aligned with the quarter circular boss such that the pair of stops engage with the pair of bayonet coupling members to provide a keyed connection; and/or
wherein a seal (126A, 126B, 136A, 136B) is disposed within the circular recess around each one of the plurality of input ports disposed therein;
optionally wherein the seal is an O-ring or a stadium shaped seal.

10. The injection needle assembly according to claim 8,
wherein the at least one side port in the mixing chamber at the distal portion of the elongate hollow stylet comprises at least three side ports in the distal portion arranged around a perimeter of the elongate hollow stylet; and/or
wherein the at least one side port in the mixing chamber at the distal portion of the elongate hollow stylet includes at least two longitudinally spaced side ports; and/or
wherein the distal end of the elongate hollow stylet is a closed distal end; and/or
wherein the distal end of the elongate hollow stylet is a closed needle tip that terminates a distal extent of the inner lumen and the outer lumen.

11. The injection needle assembly according to any one of claims 8-10,
wherein the elongate hollow stylet further comprises an elongate cannula (150) that defines the outer side wall and the outer lumen; and a stylet needle tip (135) defined by the distal portion, wherein the stylet needle tip is attached to the elongate cannula to distally close the outer lumen of the elongate cannula;
optionally wherein the inner lumen is fluidly coupled to the second one of the plurality of input ports via an angled flow channel through a body of the hub; or
optionally wherein the inner lumen is fluidly coupled to the second one of the plurality of input ports via a flow channel that extends laterally through a body of the hub.

12. A sealant delivery system (100), comprising:
a sealant applicator (110) comprising two chambers (112A, 112B) separate from one another, each chamber comprising at least one output port (112A-1, 112B-1) on a distal end thereof;
a quarter turn connector (300, 700) disposed on a distal end (111-2) of the sealant applicator adjacent to the at least one output port of each chamber; and
an injection needle assembly (13) according to claim 8, wherein, when the hub quarter turn connector interlocks with the quarter turn connector disposed on the distal end of the sealant applicator, the plurality of input ports are each aligned and sealed with one of the at least one output port of each chamber.

13. The sealant delivery system according to claim 12, wherein the quarter turn connector (700) of the sealant applicator comprises:
a circular protrusion (302) extending distally from the distal end of the sealant applicator, the circular protrusion comprising the at least one output port of each of the two chambers;
a semi-circular channel (304) disposed within the distal end of the sealant applicator along a periphery of the circular protrusion; and
a pair of bayonet coupling members (306A, 306B) disposed radially outward of the circular protrusion and the semi-circular channel.

14. The sealant delivery system according to claim 12, wherein the corresponding quarter turn connector of the injection needle assembly further comprises a pair of stops (406) extending distally from the ramped lip and aligned such that the pair of stops contact the pair of bayonet coupling members to hinder rotational movement of the corresponding quarter turn connector relative to the quarter turn connector beyond a quarter rotation.

15. The sealant delivery system according to claim 12 or claim 14, wherein a seal (126A, 126B, 136A, 136B) is disposed within the circular recess around each one of the plurality of input ports disposed therein;
optionally wherein the seal is an O-ring or a stadium shaped seal.

## Patentansprüche

1. Siegelmittel-Applikator (110), umfassend:
zwei voneinander getrennte Kammern (112A, 112B), wobei jede Kammer mindestens einen Ausgangsport (112A-1, 112B-1) an einem distalen Ende davon umfasst; und
einen Vierteldrehverbinder (300, 700), der an einem distalen Ende (111-2) des Siegelmittel-Applikators mindestens einem Ausgangsport jeder Kammer benachbart angeordnet ist, wobei der Vierteldrehverbinder so geformt ist, dass er lösbar mit einem entsprechenden Vierteldrehverbinder (450) einer Injektionsnadelanordnung (130), die eine Vielzahl von Eingangsports (132A-1, 132B-1) umfasst, oder mit einem entsprechenden Vierteldrehverbinder (400) einer Zweikammer-Mischspritze (120), die eine Vielzahl von Mischports (122A-1, 122B-1) umfasst, ineinandergreift, wobei bei Kopplung der Injektionsnadelanordnung oder der Zweikammer-Mischspritze mit dem Siegelmittel-Applikator über den Vierteldrehverbinder die Vielzahl der Eingangsports oder die Vielzahl der Mischports mit dem mindestens einen Ausgangsport jeder Kammer des Siegelmittel-Applikators ausgerichtet und abgedichtet sind;
wobei der Vierteldrehverbinder Folgendes umfasst:
einen kreisförmigen Vorsprung (302), der sich distal von dem distalen Ende des Siegelmittel-Applikators erstreckt, wobei der kreisförmige Vorsprung mindestens einen Ausgangsport von jeder der beiden Kammern umfasst;
einen halbkreisförmigen Kanal (304), der innerhalb des distalen Endes des Siegelmittel-Applikators entlang eines Umfangs des kreisförmigen Vorsprungs angeordnet ist; und
ein Paar von Bajonett-Kopplungselementen (306A, 306B), die radial außerhalb des kreisförmigen Vorsprungs und des halbkreisförmigen Kanals angeordnet sind.

2. Siegelmittel-Abgabesystem (100), umfassend einen Siegelmittel-Applikator (110) nach Anspruch 1.

3. Siegelmittel-Abgabesystem nach Anspruch 2,
wobei der entsprechende Vierteldrehverbinder (450) der Injektionsnadelanordnung und der entsprechende Vierteldrehanschluss der Zweikammer-Mischspritze jeweils Folgendes umfassen:
eine kreisförmige Aussparung (402), die so bemessen ist, dass sie den kreisförmigen Vorsprung am distalen Ende des Siegelmittel-Applikators aufnimmt, wobei die kreisförmige Aussparung die Vielzahl von Eingangsports oder die Vielzahl von Mischports umfasst, die darin angeordnet sind;
eine abgeschrägte Lippe (404), die sich von der kreisförmigen Aussparung radial nach außen erstreckt, wobei die abgeschrägte Lippe so geformt und bemessen ist, dass sie von dem Paar von Bajonett-Kopplungselementen aufgenommen und gehalten wird, wenn der entsprechende Vierteldrehverbinder relativ zu dem Vierteldrehverbinder gedreht wird; und
eine viertelkreisförmige Verlängerung (456), die sich proximal von der Lippe erstreckt, wobei die viertelkreisförmige Verlängerung so geformt ist, dass sie in dem halbkreisförmigen Kanal aufgenommen wird und innerhalb des halbkreisförmigen Kanals in einer Vierteldrehbewegung von einem entriegelten Zustand in einen verriegelten Zustand gleiten kann;
gegebenenfalls wobei der entsprechende Vierteldrehverbinder der Injektionsnadelanordnung und der entsprechende Vierteldrehverbinder der Zweikammer-Mischspritze jeweils weiter ein Paar von Anschlägen (406) umfassen, die sich distal von der Rampenlippe erstrecken und so ausgerichtet sind, dass das Paar von Anschlägen in Kontakt mit dem Paar von Bajonett-Kopplungselementen ist, um eine Drehbewegung des entsprechenden Vierteldrehverbinders relativ zum Vierteldrehverbinder über eine Vierteldrehung hinaus zu verhindern.

4. Siegelmittel-Abgabesystem nach Anspruch 3, wobei eine Dichtung (126A, 126B, 136A, 136B) innerhalb der kreisförmigen Aussparung um jeden der Vielzahl von Eingangsports oder der Vielzahl von darin angeordneten Mischports angeordnet ist;
gegebenenfalls wobei die Dichtung ein O-Ring oder eine stadionförmige Dichtung ist.

5. Siegelmittel-Abgabesystem nach einem der Ansprüche 2 bis 4, weiter umfassend:
die Injektionsnadelanordnung, wobei die Injektionsnadelanordnung Folgendes umfasst:
eine Nabe (131), die den entsprechenden Vierteldrehverbinder und die mehreren Eingangsports umfasst; und
ein längliches hohles Stilett (132), das sich distal von der Nabe erstreckt, wobei das längliche hohle Stilett einen proximalen Abschnitt an der Nabe und einen distalen Abschnitt aufweist, der von dem proximalen Abschnitt beabstandet ist.

6. Siegelmittel-Abgabesystem nach Anspruch 5, wobei das längliche hohle Stilett Folgendes umfasst:
eine äußere Seitenwand (152), die sich von dem proximalen Abschnitt zu dem distalen Abschnitt erstreckt und ein äußeres Lumen (153) definiert, das mit mindestens einem ersten der Vielzahl von Eingangsports fluidgekoppelt ist;
eine innere Seitenwand (154), die sich von dem proximalen Abschnitt zu dem distalen Abschnitt erstreckt und ein inneres Lumen (155) definiert, das innerhalb des äußeren Lumens so angeordnet ist, dass das innere Lumen konzentrisch zu dem äußeren Lumen ist und eine Querschnittsgröße aufweist, die kleiner ist als das äußere Lumen, wobei das innere Lumen mit mindestens einem zweiten der mehreren Eingangsports fluidgekoppelt ist; und
eine Mischkammer (151), die am distalen Abschnitt des länglichen hohlen Stiletts angeordnet ist, wobei die Mischkammer mit dem äußeren Lumen und dem inneren Lumen fluidgekoppelt ist und mindestens einen Seitenport (134A, 134B) umfasst.

7. Siegelmittel-Abgabesystem nach einem der Ansprüche 2 bis 6, weiter umfassend:
die Zweikammer-Mischspritze, wobei die Zweikammer-Mischspritze Folgendes umfasst:
eine erste Mischkammer (122A), die einen ersten Mischport (122A-1) der Vielzahl von Mischports aufweist, und
eine zweite Mischkammer (122B), die einen zweiten Mischport (122B-1) der Vielzahl von Mischports aufweist.

8. Injektionsnadelanordnung, umfassend:
eine Nabe (131), die einen Naben-Vierteldrehverbinder (450) aufweist, der so ausgelegt ist, dass er lösbar mit einem entsprechenden Vierteldrehverbinder verriegelt, der an einem Siegelmittel-Applikator angeordnet ist;
eine Vielzahl von Eingangsports (132A-1, 132B-1), die innerhalb der Nabe angeordnet sind; und
ein längliches hohles Stilett (132), das sich distal von der Nabe erstreckt, wobei das längliche hohle Stilett einen proximalen Abschnitt an der Nabe und einen distalen Abschnitt aufweist, der von dem proximalen Abschnitt beabstandet ist, wobei das längliche hohle Stilett Folgendes umfasst:
eine äußere Seitenwand (152), die sich von dem proximalen Abschnitt zu dem distalen Abschnitt erstreckt und ein äußeres Lumen (153) definiert, das mit mindestens einem ersten der Vielzahl von Eingangsports fluidgekoppelt ist,
eine innere Seitenwand (154), die sich von dem proximalen Abschnitt zu dem distalen Abschnitt erstreckt und ein inneres Lumen (155) definiert, das innerhalb des äußeren Lumens so angeordnet ist, dass das innere Lumen konzentrisch zu dem äußeren Lumen ist und eine Querschnittsgröße aufweist, die kleiner ist als das äußere Lumen, wobei das innere Lumen mit mindestens einem zweiten der mehreren Eingangsports fluidgekoppelt ist, und
eine Mischkammer (151), die am distalen Abschnitt des länglichen hohlen Stiletts angeordnet ist, wobei die Mischkammer mit dem äußeren Lumen und dem inneren Lumen fluidgekoppelt ist und mindestens einen Seitenport umfasst,
wobei, wenn der Naben-Vierteldrehverbinder mit dem an einem distalen Ende des Siegelmittel-Applikators angeordneten Vierteldrehverbinder ineinandergreift, die Vielzahl von Eingangsports jeweils mit einem entsprechenden Ausgangsport des Siegelmittel-Applikators ausgerichtet und abgedichtet sind;
wobei der Naben-Vierteldrehverbinder Folgendes umfasst:
eine kreisförmige Aussparung (402), die so bemessen ist, dass sie einen kreisförmigen Vorsprung am distalen Ende des Siegelmittel-Applikators aufnimmt, wobei die kreisförmige Aussparung die Vielzahl von Eingangsports umfasst, die darin angeordnet sind;
eine Lippe (402), die sich radial nach außen von der kreisförmigen Aussparung erstreckt, wobei die Lippe so geformt und bemessen ist, dass sie in einem Paar Bajonett-Kopplungselementen aufgenommen werden kann, die an dem Siegelmittel-Applikator angeordnet sind; und
eine viertelkreisförmige Nase (456), die sich proximal von der Lippe erstreckt, wobei die viertelkreisförmige Nase so geformt ist, dass sie in einen halbkreisförmigen Kanal des Siegelmittel-Applikators aufgenommen wird und innerhalb des halbkreisförmigen Kanals in einer Vierteldrehbewegung von einem entriegelten Zustand in einen verriegelten Zustand gleiten kann.

9. Injektionsnadelanordnung nach Anspruch 8,
wobei der Naben-Vierteldrehverbinder weiter ein Paar Anschläge (406) umfasst, die sich distal von der Lippe erstrecken und mit dem viertelkreisförmigen Vorsprung ausgerichtet sind so dass das Paar Anschläge mit dem Paar Bajonett-Kopplungselementen eingreift, um eine Keilverbindung bereitzustellen; und/oder
wobei eine Dichtung (126A, 126B, 136A, 136B) innerhalb der kreisförmigen Aussparung um jeden der Vielzahl von darin angeordneten Eingangsports angeordnet ist;
gegebenenfalls wobei die Dichtung ein O-Ring oder eine stadionförmige Dichtung ist.

10. Injektionsnadelanordnung nach Anspruch 8,
wobei der mindestens eine Seitenport in der Mischkammer am distalen Abschnitt des länglichen hohlen Stiletts mindestens drei Seitenports im distalen Abschnitt umfasst, die um einen Umfang des länglichen hohlen Stiletts herum angeordnet sind; und/oder
wobei der mindestens eine Seitenport in der Mischkammer am distalen Abschnitt des länglichen Stiletts mindestens zwei in Längsrichtung beabstandete Seitenports schließt ein; und/oder
wobei das distale Ende des länglichen hohlen Stiletts ein geschlossenes distales Ende ist; und/oder
wobei das distale Ende des länglichen hohlen Stiletts eine geschlossene Nadelspitze ist, die einen distalen Abschnitt des inneren Lumens und des äußeren Lumens beendet.

11. Injektionsnadelanordnung nach einem der Ansprüche 8 bis 10, wobei
das längliche hohle Stilett weiter eine längliche Kanüle (150) umfasst, die die äußere Seitenwand und das äußere Lumen definiert; und eine Stilettnadelspitze (135), die durch den distalen Abschnitt definiert ist, wobei die Stilettnadelspitze an der länglichen Kanüle befestigt ist, um das äußere Lumen der länglichen Kanüle distal zu verschließen;
gegebenenfalls wobei das innere Lumen über einen abgewinkelten Strömungskanal durch einen Körper der Nabe mit dem zweiten der Vielzahl von Eingangsports fluidgekoppelt ist; oder
gegebenenfalls wobei das innere Lumen über einen Strömungskanal, der sich seitlich durch einen Körper der Nabe erstreckt, mit dem zweiten der Vielzahl von Eingangsports fluidgekoppelt ist.

12. Siegelmittel-Abgabesystem (100), umfassend:
einen Siegelmittel-Applikator (110), umfassend zwei voneinander getrennte Kammern (112A, 112B), wobei jede Kammer mindestens einen Ausgangsport (112A-1, 112B-1) an einem distalen Ende davon umfasst;
einen Vierteldrehverbinder (300, 700), der an einem distalen Ende (111-2) des Siegelmittel-Applikators mindestens einem Ausgangsport jeder Kammer benachbart angeordnet ist; und
eine Injektionsnadelanordnung (13) nach Anspruch 8, wobei, wenn der Naben-Vierteldrehverbinder mit dem an dem distalen Ende des Siegelmittel-Applikators angeordneten Vierteldrehverbinder ineinandergreift, die Vielzahl von Eingangsports jeweils mit einem der mindestens einen Ausgangsport jeder Kammer ausgerichtet und abgedichtet sind.

13. Siegelmittel-Abgabesystem nach Anspruch 12, wobei der Vierteldrehverbinder (700) des Siegelmittel-Applikators Folgendes umfasst:
einen kreisförmigen Vorsprung (302), der sich distal von dem distalen Ende des Siegelmittel-Applikators erstreckt, wobei der kreisförmige Vorsprung mindestens einen Ausgangsport von jeder der beiden Kammern umfasst;
einen halbkreisförmigen Kanal (304), der innerhalb des distalen Endes des Siegelmittel-Applikators entlang eines Umfangs des kreisförmigen Vorsprungs angeordnet ist; und
ein Paar von Bajonett-Kopplungselementen (306A, 306B), die radial außerhalb des kreisförmigen Vorsprungs und des halbkreisförmigen Kanals angeordnet sind.

14. Siegelmittel-Abgabesystem nach Anspruch 12, wobei der entsprechende Vierteldrehverbinder der Injektionsnadelanordnung weiter ein Paar Anschläge (406) umfasst, die sich distal von der abgeschrägten Lippe erstrecken und so ausgerichtet sind, dass das Paar von Anschlägen in Kontakt mit dem Paar von Bajonett-Kopplungselementen ist, um eine Drehbewegung des entsprechenden Vierteldrehverbinders relativ zum Vierteldrehverbinder über eine Vierteldrehung hinaus zu verhindern.

15. Siegelmittel-Abgabesystem nach Anspruch 12 oder Anspruch 14, wobei eine Dichtung (126A, 126B, 136A, 136B) innerhalb der kreisförmigen Aussparung um jeden der Vielzahl von darin angeordneten Eingangsports angeordnet ist;
gegebenenfalls wobei die Dichtung ein O-Ring oder eine stadionförmige Dichtung ist.

## Revendications

1. Applicateur (110) de produit d'étanchéité, comprenant :
deux chambres (112A, 112B) séparées l'une de l'autre, chaque chambre comprenant au moins un orifice de sortie (112A-1, 112B-1) sur une extrémité distale de celle-ci ; et
un raccord quart de tour (300, 700) disposé sur une extrémité distale (111-2) de l'applicateur de produit d'étanchéité de manière adjacente à l'au moins un orifice de sortie de chaque chambre, le raccord quart de tour formé pour s'emboîter de manière détachable avec un raccord quart de tour (450) correspondant d'un ensemble d'aiguille d'injection (130) comprenant une pluralité d'orifices d'entrée (132A-1, 132B-1) ou avec un raccord quart de tour (400) correspondant d'une seringue de mélange à double chambre (120) comprenant une pluralité d'orifices de mélange (122A-1, 122B-1), dans lequel, lorsque l'ensemble d'aiguille d'injection ou la seringue de mélange à double chambre est couplé(e) à l'applicateur de produit d'étanchéité via le raccord quart de tour, la pluralité d'orifices d'entrée ou la pluralité d'orifices de mélange sont alignés et scellés avec l'au moins un orifice de sortie de chaque chambre de l'applicateur de produit d'étanchéité ;
dans lequel le raccord quart de tour comprend :
une saillie circulaire (302) s'étendant distalement à partir de l'extrémité distale de l'applicateur de produit d'étanchéité, la saillie circulaire comprenant l'au moins un orifice de sortie de chacune des deux chambres ;
un canal semi-circulaire (304) disposé au sein de l'extrémité distale de l'applicateur de produit d'étanchéité le long d'une périphérie de la saillie circulaire ; et
une paire d'organes de couplage à baïonnette (306A, 306B) disposés radialement vers l'extérieur de la saillie circulaire et du canal semi-circulaire.

2. Système (100) de distribution de produit d'étanchéité, comprenant un applicateur (110) de produit d'étanchéité selon la revendication 1.

3. Système de distribution de produit d'étanchéité selon la revendication 2,
dans lequel le raccord quart de tour (450) correspondant de l'ensemble d'aiguille d'injection et le raccord quart de tour correspondant de la seringue de mélange à double chambre comprennent chacun :
un évidement circulaire (402) dimensionné pour recevoir la saillie circulaire sur l'extrémité distale de l'applicateur de produit d'étanchéité, l'évidement circulaire comprenant la pluralité d'orifices d'entrée ou la pluralité d'orifices de mélange disposés dans celui-ci ;
une lèvre inclinée (404) s'étendant radialement vers l'extérieur de l'évidement circulaire, la lèvre inclinée étant formée et dimensionnée pour être reçue et retenue par la paire d'organes de couplage à baïonnette lorsque le raccord quart de tour correspondant est tourné par rapport au raccord quart de tour ; et
une extension quart circulaire (456) s'étendant proximalement à partir de la lèvre, l'extension quart circulaire étant formée pour être reçue au sein du canal semi-circulaire et coulissante au sein du canal semi-circulaire dans un mouvement d'un quart de tour d'un état déverrouillé à un état verrouillé ;
facultativement dans lequel le raccord quart de tour correspondant de l'ensemble d'aiguille d'injection et le raccord quart de tour correspondant de la seringue de mélange à double chambre comprennent en outre chacun une paire de butées (406) s'étendant distalement à partir de la lèvre inclinée et alignées de sorte que la paire de butées entre en contact avec la paire d'organes de couplage à baïonnette pour empêcher le mouvement de rotation du raccord quart de tour correspondant par rapport au raccord quart de tour au-delà d'un quart de tour.

4. Système de distribution de produit d'étanchéité selon la revendication 3, dans lequel un joint (126A, 126B, 136A, 136B) est disposé au sein de l'évidement circulaire autour de chacun de la pluralité d'orifices d'entrée ou de la pluralité d'orifices de mélange disposés à l'intérieur ;
facultativement dans lequel le joint est un joint torique ou un joint en forme de stade.

5. Système de distribution de produit d'étanchéité selon l'une quelconque des revendications 2 à 4, comprenant en outre :
l'ensemble d'aiguille d'injection, l'ensemble d'aiguille d'injection comprenant :
une embase (131) comprenant le raccord quart de tour correspondant et la pluralité de ports d'entrée ; et
un stylet creux allongé (132) qui s'étend distalement à partir de l'embase, le stylet creux allongé ayant une portion proximale au niveau de l'embase et une portion distale espacée de la portion proximale.

6. Système de distribution de produit d'étanchéité selon la revendication 5, dans lequel le stylet creux allongé comprend :
une paroi latérale externe (152) s'étendant de la portion proximale à la portion distale et définissant une lumière externe (153) qui est couplée fluidiquement à au moins un premier de la pluralité d'orifices d'entrée ;
une paroi latérale interne (154) s'étendant de la portion proximale à la portion distale et définissant une lumière interne (155) disposée au sein de la lumière externe de sorte que la lumière interne soit concentrique avec la lumière externe et ait une taille de section transversale qui est plus petite que la lumière externe, la lumière interne étant couplée fluidiquement à au moins un deuxième de la pluralité d'orifices d'entrée ; et
une chambre de mélange (151) disposée au niveau de la portion distale du stylet creux allongé, la chambre de mélange étant couplée fluidiquement à la lumière externe et à la lumière interne et comprenant au moins un orifice latéral (134A, 134B).

7. Système de distribution de produit d'étanchéité selon l'une quelconque des revendications 2 à 6, comprenant en outre :
la seringue de mélange à double chambre, la seringue de mélange à double chambre comprenant :
une première chambre de mélange (122A) ayant un premier orifice de mélange (122A-1) de la pluralité d'orifices de mélange, et
une deuxième chambre de mélange (122B) ayant un deuxième orifice de mélange (122B-1) de la pluralité d'orifices de mélange.

8. Ensemble d'aiguille d'injection, comprenant :
une embase (131) ayant un raccord quart de tour d'embase (450) qui est configuré pour s'emboîter de manière détachable avec un raccord quart de tour correspondant disposé sur un applicateur de produit d'étanchéité ;
une pluralité d'orifices d'entrée (132A-1, 132B-1) disposés au sein de l'embase ; et
un stylet creux allongé (132) qui s'étend distalement à partir de l'embase, le stylet creux allongé ayant une portion proximale au niveau de l'embase et une portion distale espacée de la portion proximale, le stylet creux allongé comprenant :
une paroi latérale externe (152) s'étendant de la portion proximale à la portion distale et définissant une lumière externe (153) qui est couplée fluidiquement à au moins un premier de la pluralité d'orifices d'entrée,
une paroi latérale interne (154) s'étendant de la portion proximale à la portion distale et définissant une lumière interne (155) disposée au sein de la lumière externe de sorte que la lumière interne soit concentrique avec la lumière externe et ait une taille de section transversale qui est plus petite que la lumière externe, la lumière interne étant couplée fluidiquement à au moins un deuxième de la pluralité d'orifices d'entrée, et
une chambre de mélange (151) disposée au niveau de la portion distale du stylet creux allongé, la chambre de mélange étant couplée fluidiquement à la lumière externe et à la lumière interne et comprenant au moins un orifice latéral,
dans lequel, lorsque le raccord quart de tour d'embase s'emboîte avec le raccord quart de tour disposé sur une extrémité distale de l'applicateur de produit d'étanchéité, la pluralité d'orifices d'entrée sont chacun alignés et scellés avec un orifice de sortie correspondant de l'applicateur de produit d'étanchéité ;
dans lequel le raccord quart de tour d'embase comprend :
un évidement circulaire (402) dimensionné pour recevoir une saillie circulaire sur l'extrémité distale de l'applicateur de produit d'étanchéité, l'évidement circulaire comprenant la pluralité d'orifices d'entrée disposés à l'intérieur ;
une lèvre (402) s'étendant radialement vers l'extérieur de l'évidement circulaire, la lèvre étant formée et dimensionnée pour être reçue au sein d'une paire d'organes de couplage à baïonnette disposés sur l'applicateur de produit d'étanchéité ; et
un bossage quart circulaire (456) s'étendant proximalement à partir de la lèvre, le bossage quart circulaire étant formé pour être reçu dans un canal semi-circulaire de l'applicateur de produit d'étanchéité et coulissant au sein du canal semi-circulaire dans un mouvement d'un quart de tour d'un état déverrouillé à un état verrouillé.

9. Ensemble d'aiguille d'injection selon la revendication 8,
dans lequel le raccord quart de tour d'embase comprend en outre une paire de butées (406) s'étendant distalement à partir de la lèvre et alignées avec le bossage quart circulaire de sorte que la paire de butées est en prise avec la paire d'organes de couplage à baïonnette pour fournir une connexion à clavette ; et/ou
dans lequel un joint (126A, 126B, 136A, 136B) est disposé au sein de l'évidement circulaire autour de chacun de la pluralité d'orifices d'entrée disposés à l'intérieur ;
facultativement dans lequel le joint est un joint torique ou un joint en forme de stade.

10. Ensemble d'aiguille d'injection selon la revendication 8,
dans lequel l'au moins un orifice latéral dans la chambre de mélange au niveau de la portion distale du stylet creux allongé comprend au moins trois orifices latéraux dans la portion distale agencés autour d'un périmètre du stylet creux allongé ; et/ou
dans lequel l'au moins un orifice latéral dans la chambre de mélange au niveau de la portion distale du stylet creux allongé inclut au moins deux orifices latéraux espacés longitudinalement ; et/ou
dans lequel l'extrémité distale du stylet creux allongé est une extrémité distale fermée ; et/ou
dans lequel l'extrémité distale du stylet creux allongé est une pointe d'aiguille fermée qui termine une extension distale de la lumière interne et de la lumière externe.

11. Ensemble d'aiguille d'injection selon l'une quelconque des revendications 8 à 10, dans lequel
le stylet creux allongé comprend en outre une canule allongée (150) qui définit la paroi latérale externe et la lumière externe ; et une pointe d'aiguille de stylet (135) définie par la portion distale, dans lequel la pointe d'aiguille de stylet est fixée à la canule allongée pour fermer distalement la lumière externe de la canule allongée ;
facultativement dans lequel la lumière interne est couplée fluidiquement au deuxième de la pluralité d'orifices d'entrée via un canal d'écoulement en biais à travers un corps de l'embase ; ou
facultativement dans lequel la lumière interne est couplée fluidiquement au deuxième de la pluralité d'orifices d'entrée via un canal d'écoulement qui s'étend latéralement à travers un corps de l'embase.

12. Système (100) de distribution de produit d'étanchéité, comprenant :
un applicateur (110) de produit d'étanchéité comprenant deux chambres (112A, 112B) séparées l'une de l'autre, chaque chambre comprenant au moins un orifice de sortie (112A-1, 112B-1) sur une extrémité distale de celle-ci ;
un raccord quart de tour (300, 700) disposé sur une extrémité distale (111-2) de l'applicateur de produit d'étanchéité de manière adjacente à l'au moins un orifice de sortie de chaque chambre ; et
un ensemble d'aiguille d'injection (13) selon la revendication 8, dans lequel, lorsque le raccord quart de tour d'embase s'emboîte avec le raccord quart de tour disposé sur l'extrémité distale de l'applicateur de produit d'étanchéité, la pluralité d'orifices d'entrée sont chacun alignés et scellés avec l'un de l'au moins un orifice de sortie de chaque chambre.

13. Système de distribution de produit d'étanchéité selon la revendication 12, dans lequel le raccord quart de tour (700) de l'applicateur de produit d'étanchéité comprend :
une saillie circulaire (302) s'étendant distalement à partir de l'extrémité distale de l'applicateur de produit d'étanchéité, la saillie circulaire comprenant l'au moins un orifice de sortie de chacune des deux chambres ;
un canal semi-circulaire (304) disposé au sein de l'extrémité distale de l'applicateur de produit d'étanchéité le long d'une périphérie de la saillie circulaire ; et
une paire d'organes de couplage à baïonnette (306A, 306B) disposés radialement vers l'extérieur de la saillie circulaire et du canal semi-circulaire.

14. Système de distribution de produit d'étanchéité selon la revendication 12, dans lequel le raccord quart de tour correspondant de l'ensemble d'aiguille d'injection comprend en outre une paire de butées (406) s'étendant distalement à partir de la lèvre inclinée et alignées de sorte que la paire de butées entre en contact avec la paire d'organes de couplage à baïonnette pour empêcher le mouvement de rotation du raccord quart de tour correspondant par rapport au raccord quart de tour au-delà d'un quart de tour.

15. Système de distribution de produit d'étanchéité selon la revendication 12 ou la revendication 14, dans lequel un joint (126A, 126B, 136A, 136B) est disposé au sein de l'évidement circulaire autour de chacun de la pluralité d'orifices d'entrée disposés à l'intérieur ;
facultativement dans lequel le joint est un joint torique ou un joint en forme de stade.
